(19) **Europäisches Patentamt** / **European Patent Office** / **Office européen des brevets**

(11) **EP 3 844 270 B1**

(12) **EUROPEAN PATENT SPECIFICATION**

(45) Date of publication and mention of the grant of the patent:
**12.07.2023 Bulletin 2023/28**

(21) Application number: **19769998.6**

(22) Date of filing: **30.08.2019**

(51) International Patent Classification (IPC):
*C12N 9/12* *(2006.01)* *G01N 33/542* *(2006.01)*

(52) Cooperative Patent Classification (CPC):
**C12Y 207/01037; C12N 9/1205;** C07K 2319/60;
G01N 33/542

(86) International application number:
**PCT/EP2019/073179**

(87) International publication number:
**WO 2020/043867 (05.03.2020 Gazette 2020/10)**

(54) **CALCIUM DEPENDENT PROTEIN KINASE CONSTRUCTS AND USES THEREOF**

KALZIUM-ABHÄNGIGE PROTEIN KINASE-KONSTRUKTE UND DEREN VERWENDUNG

CONSTRUCTIONS DE PROTEINE KINASE DEPENDANTE DU CALCIUM ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.08.2018 EP 18191949**

(43) Date of publication of application:
**07.07.2021 Bulletin 2021/27**

(73) Proprietor: **Leibniz-Institut für Pflanzenbiochemie (IPB)**
**06120 Halle (Saale) (DE)**

(72) Inventors:
• **ROMEIS, Tina**
**06114 Halle (Saale) (DE)**
• **LIESE, Anja**
**06120 Halle (Saale) (DE)**

(74) Representative: **Schiweck Weinzierl Koch Patentanwälte Partnerschaft mbB**
**Ganghoferstraße 68 B**
**80339 München (DE)**

(56) References cited:
• **DEPRY, C: "Using FRET-Based Reporters to Visualize Subcellular Dynamics of Protein Kinase A Activity", METHODS MOL BIOL, vol. 756, 2011, pages 285-294, XP009508651,**
• **ALLEN M D ET AL: "Subcellular dynamics of protein kinase A activity visualized by FRET-based reporters", BIOCHEMICAL AND BIOPHYSICAL RESEARCH COMMUNICATIONS, ELSEVIER, AMSTERDAM, NL, vol. 348, no. 2, 22 September 2006 (2006-09-22), pages 716-721, XP024924287, ISSN: 0006-291X, DOI: 10.1016/J.BBRC.2006.07.136 [retrieved on 2006-09-22]**
• **RODEMS S M ET AL: "A FRET-BASED ASSAY PLATFORM FOR ULTRA-HIGH DENSITY DRUG SCREENING OF PROTEIN KINASES AND PHOSPHATASES", ASSAY AND DRUG DEVELOPMENT TECHNOLOGIES, MARY ANN LIEBERT, INC. PUBLISHERS, US, vol. 1, no. 1, PART 01, 1 November 2002 (2002-11-01), pages 9-19, XP001183957, ISSN: 1540-658X, DOI: 10.1089/154065802761001266**

Remarks:
The complete document including Reference Table(s) and the Sequence Listing(s) can be downloaded from the EPO website

EP 3 844 270 B1

**Description**

The present invention relates to calcium dependent protein kinase (CDPK) constructs comprising a pair of chromophores suitable for measuring real-time FRET occurrence upon conformational changes or activation of the CDPK construct. Particularly, the present invention relates to CDPK polypeptides comprising a variable domain (VD), a protein kinase domain (PKD), a pseudosubstrate segment (PS), and a calmodulin-like domain (CLD), said CLD comprising 4 EF-hand motifs EF1, EF2, EF3 and EF4, wherein a donor chromophore is inserted between PKD and PS and an acceptor chromophore is inserted C-terminally of EF4, or an acceptor chromophore is inserted between PKD and PS and a donor chromophore is inserted C-terminally of EF4, wherein said donor chromophore and said acceptor chromophore represent a Förster Resonance Energy Transfer (FRET) pair. The present invention further relates to polynucleotides encoding such polypeptides as well as vectors and host cells comprising such polypeptides and/or polynucleotides. Furthermore, the present invention relates to methods for measuring conformational change or activation status of a CDPK polypeptide by employing said polypeptides and to uses of said polypeptides for measuring conformational change or activation status of a CDPK polypeptide.

In most eukaryotic signal transduction pathways, calcium ($Ca^{2+}$) signatures are translated in phosphorylation cascades. Calcium-dependent protein kinases (CDPKs), enzymes specific to plants and important human parasites, bind $Ca^{2+}$ directly and transduce the $Ca^{2+}$-signal into protein phosphorylation (Kudla et al., New Phytol (2018), doi:10.1 111/nph.14966; Schulz et al., Plant Physiol (2013) 163: 523-530). In plants, the CDPK gene family is implicated in abiotic and biotic stress as well as developmental signaling (Geiger et al., Sci Signal (2011), 4: ra32, doi:10.1126/scisignal.2001346; Geiger et al., Proc Natl Acad Sci U S A (2010), 107: 8023-8028; Dubiella et al., Proc Natl Acad Sci USA (2013), 110: 8744-8749; Boudsocq et al., Nature (2010), 464: 418-422, doi:10.1038/nature08794; Liu, et al., Nature (2017), 545: 311-316; Gutermuth et al., New Phytologist (2018), 218: 1089-1105; Gutermuth et al., Plant Cell (2013), 25: 4525-4543). CDPKs from protists including human parasites such as Plasmodium falciparum are important for the parasitic life cycle (Foroutan et al., Clin Exp Vaccine Res (2018), 7: 24-36; Ojo et al., , Nat Struct Mol Biol (2010), 17: 602-607; Billker et al., Cell Host Microbe (2009), 5: 612-622; Kato et al., Nat Chem Biol (2008), 4: 347-356) and have been considered targets for drug design and vaccination (Foroutan, loc. cit., Ojo, loc. cit.).

$Ca^{2+}$-dependent activation of calcium dependent protein kinases (CDPKs, also referred to as C(D)PK or CPK in *Arabidopsis thaliana)* is determined through the conserved modular protein structure, consisting of an N-terminal variable domain, with some members carrying myristoylation and palmitoylation membrane-localization motifs (Simeunovic et al., J Exp Bot (2016), 67: 3855-3872), a serine/threonine protein kinase domain, and a pseudosubstrate segment (PS), as well as a calmodulin-like domain (CLD) containing four $Ca^{2+}$-binding EF-hand motifs (Kudla, loc. cit.; Schulz, loc. cit.; Liese et al., Biochim Biophys Acta (2013), 1833, 1582-1589; Bender et al., Biochem J (2018), 475: 207).

CPK21 and its closest homologue CPK23 have overlapping functions in abscisic acid (ABA) signal transduction activating the same target anion channels in stomata (Geiger (2010), loc. cit.; Geiger (2011), loc. cit.). However, $Ca^{2+}$-sensitive anion channel activation was assigned to the CPK21 pathway because CPK23 turned out to be rather $Ca^{2+}$-insensitive (Geiger (2010), loc. cit.; Geiger (2011), loc. cit.).

At basal $Ca^{2+}$-levels, CDPKs are kept inactive via interactions between the kinase domain and the PS. $Ca^{2+}$-binding to the CLD induces an open and active conformation (Wernimont et al., Nat Struct Mol Biol (2010), 17: 596-601; Wernimont et al., Proteins (2011), 79: 803-820). A detailed investigation of temporal and spatial activation of CDPKs in the context of plant signal transduction or parasitic infection has been hampered by the lack of appropriate *in vivo* approaches.

The present invention addresses these needs and objectives by providing solutions as described herein and as defined in the claims.

In accordance with the present invention, a ratiometric Förster resonance energy transfer (FRET)-based reporter was constructed, enabling *in vitro* and *in vivo* CDPK conformational change measurement over a wide range of natural $Ca^{2+}$ concentrations. As found in context with the present invention, the exemplary investigation of two CDPKs (also referred to herein as C(D)PK or CPK, particularly in context with *Arabidopsis thaliana)* from *Arabidopsis thaliana* with distinct $Ca^{2+}$-affinities demonstrated the impact of single amino acid residues on $Ca^{2+}$-binding dynamics. Furthermore, in context with the present invention it could be shown that half maximal effective concentration (EC50) for $Ca^{2+}$-dependent conformational change mirrors the half maximal activity (K50) for $Ca^{2+}$-dependent CDPK enzyme activity. In the life cell system plant pollen tubes, FRET sensor-based imaging of the kinase conformational change records genuine $Ca^{2+}$-dependent enzyme activation and inactivation in real time. The CDPK-FRET according to the present invention was found to be highly suitable and effective tool for functional studies of CDPKs in sub-cellular temporal and spatial resolution within multiple signal transduction pathways in both plants and protists.

Thus, the present invention relates to a calcium dependent protein kinase (referred to herein as CDPK, CPK or C(D)PK) polypeptide comprising a variable domain (VD), a protein kinase domain (PKD), a pseudosubstrate segment (PS), and a calmodulin-like domain (CLD), said CLD comprising 4 EF-hand motifs EF1, EF2, EF3 and EF4, wherein

(A) a donor chromophore is inserted between PKD and PS and an acceptor chromophore is inserted C-terminally of EF4, or

(B) an acceptor chromophore is inserted between PKD and PS and a donor chromophore is inserted C-terminally of EF4,

wherein said donor chromophore and said acceptor chromophore represent a Förster Resonance Energy Transfer (FRET) pair.

As known in the art and as used herein, a calcium dependent protein kinase (CDPK, CPK) comprises or consists of an N-terminal variable domain (VD), with some members carrying myristoylation and palmitoylation membrane-localization motifs (Simeunovic, loc. cit.), a serine/threonine protein kinase domain (PKD), a pseudosubstrate segment (PS), as well as a calmodulin-like domain (CLD) containing four $Ca^{2+}$-binding EF-hand motifs EF1, EF2, EF3 and EF4 (Kudla, loc. cit.; Schulz, loc. cit.; Liese, loc. cit.; Bender, loc. cit.). The wellconserved structure of C(D)PKs is also known in the art and described in, e.g., Hamel et al., Trends Plant Sci (2014), 19(2): 79-89.

In context with the present invention, a FRET approach was performed where the PS and CLD of CDPKs was sandwiched between a donor (e.g., mTurquoise, mT) chromophore and an acceptor (e.g., Venus circularly permuted at amino acid 173, cpV173) chromophore. In accordance with the present invention, the FRET donor is inserted between PKD and PS because the kinase domain stabilizes the inactive enzyme conformation via interaction with the PS.

The present invention provides the first FRET-based reporter that detects CDPK conformational changes indicative of enzyme activation. In contrast to substrate-based FRET reporters where typically a substrate is sandwiched between a fluorescence protein pair

(Depry et al., Methods Mol Biol 2011; (756: 285-294), in context with the present invention intrinsic intramolecular $Ca^{2+}$-dependent conformational changes of the enzyme itself correlating with enzyme activation were monitored. The inventive C(D)PK polypeptide described and provided herein provides furthermore an independent tool for the identification of amino acid residues which are essential for the enzyme-specific decoding capacity of changes in cytoplasmic $[Ca^{2+}]$.

With CDPKs being absent in human and animal cells, the C(D)PK polypeptide of the present invention also overcomes a limitation of calmodulin-based $Ca^{2+}$-sensors of interfering with the host cell biochemistry. Therefore, the C(D)PK polypeptide of the present invention also provides a novel type of $Ca^{2+}$-sensors applicable to a wide range of cytoplasmic $Ca^{2+}$ concentrations and to multiple functional *in vivo* contexts useful for non-plant research of $Ca^{2+}$-dependent signaling or CDPK-based drug design.

In accordance with the present invention, in a preferred embodiment a donor chromophore is inserted between the PKD and the PS of the C(D)PK, and an acceptor chromophore of the FRET-pair is then inserted C-terminally of the EF4 (i.e. the 4th EF hand motif of the CLD). In another embodiment of the present invention, an acceptor chromophore is inserted between the PKD and the PS of the C(D)PK, and a donor chromophore of the FRET-pair is then inserted C-terminally of the EF4 (i.e. the 4th EF hand motif of the CLD).

Generally, in accordance with the present invention, it is also possible that the donor and/or the acceptor chromophores are inserted into the respective sites of the C(D)PKs using a linker molecule. Such linker may be a short (e.g., approximately 2 - 20 amino acids, app. 2 - 10 amino acids or app. 2 - 8 amino acids) peptide chain or other linker, upstream and/or downstream of the chromophore to be inserted, suitable to insert the chromophore (preferably, a FP) as described herein into the C(D)PK.

In accordance with the present invention, sequences of exemplary CDPKs (CPKs) with its specific domains (e.g., PKD, PS, CLD, C-terminus) are described herein and known in the art; cf., e.g., Table 1. As known in the art, in most CDPKs, there is also a short variable region between the PKD and the PS, and/or a variable region C-terminally of the 4th EF hand motif of the CLD. In context with the present invention, the respective chromophores may be inserted directly adjacent to or within such variable regions between PKD and PS and/or C-terminally of the EF4. Examples of C(D)PKs comprise in accordance with the present invention CPK21, CPK23, CPK1 (all derived from *Arabidopsis thaliana)* as well as further C(D)PKs as listed in Table 1 herein. In one embodiment of the present invention, the C(D)PK is CPK21 or CPK23.

As described herein in context with the present invention, one chromophore (the donor or the acceptor chromophore of the FRET pair, preferably the donor chromophore) is inserted between the PKD and the PS. Accordingly, such chromophore may be inserted directly after (i.e. directly C-terminally of) the PKD of a C(D)PK, at any site within the variable region between the PKD and the PS, and directly before (i.e., directly N-terminally of) the PS. In one embodiment, the (acceptor or donor, preferably donor) chromophore is inserted directly N-terminally of the PS of the C(D)PK. For example, in accordance with the present invention, the (acceptor or donor, preferably donor) chromophore may be inserted at a position corresponding to the position between amino acid positions 338 and 343 of SEQ ID NO: 1 (e.g., between amino acid positions 342 and 343 of SEQ ID NO: 1), at a position corresponding to the position between amino acid positions 327 and 332 of SEQ ID NO: 2 (e.g., between amino acid positions 331 and 332 of SEQ ID NO: 2), at a position corresponding to the position between amino acid positions 408 and 414 of SEQ ID NO: 3 (e.g., between amino acid positions 413 and 414 of SEQ ID NO: 3), at a position corresponding to the position between amino acid positions 363 and 368 of SEQ ID NO: 4 (e.g., between amino acid positions 367 and 368 of SEQ ID NO: 4), at a position corresponding to the position between amino acid positions 324 and 330 of SEQ ID NO: 5 (e.g., between amino acid positions 329 and 330 of SEQ ID NO: 5), at a position corresponding to the position between amino acid positions 290 and 296 of SEQ ID NO: 6 (e.g., between amino acid positions 295 and 296 of SEQ ID NO: 6), at a position corresponding to the position between amino acid positions 287 and 293 of SEQ ID NO: 7 (e.g., between amino acid positions 292 and 293 of SEQ ID NO: 7), at a position corresponding to the position between amino acid positions 292 and 298 of SEQ ID NO: 8 (e.g., between amino acid positions 297 and 298 of SEQ ID NO: 8), at a position corresponding to the position between amino acid positions 292 and 298 of SEQ ID NO: 9 (e.g., between amino acid positions 297 and 298 of SEQ ID NO: 9), at a position corresponding to the position between amino acid positions 325 and 334 of SEQ ID NO: 10 (e.g., between amino acid positions 333 and 334 of SEQ ID NO: 10), or at a position corresponding to the position between amino acid positions 308 and 317 of SEQ ID NO: 11 (e.g., between amino acid positions 316 and 317 of SEQ ID NO: 11). In a specific embodiment of the present invention, the (acceptor or donor, preferably donor) chromophore may be inserted at a position corresponding to the position between amino acid positions 342 and 343 of SEQ ID NO: 1 or at a position corresponding to the position between amino acid positions 331 and 332 of SEQ ID NO: 2.

In accordance with the present invention, insertion of a (donor or acceptor, preferably acceptor) chromophore C-terminally of EF4 (i.e. of the 4th EF hand motif of the CLD of C(D)PK) may be directly after (i.e. C-terminally of) the EF4, or (preferably directly) C-terminally of the C-terminus of the C(D)PK polypeptide, or between the C-terminus of the EF4 of the C(D)PK and the C-terminus of the C(D)PK (in cases where there is a further variable region after the EF4 of the CLD comprised by the respective C(D)PK; cf., e.g., SEQ ID NOs. 1-10 as also described in Table 1). In one embodiment of the present invention, the donor or acceptor, preferably acceptor) chromophore is inserted directly after (i.e. C-terminally of) the EF4. For example, in accordance with the present invention, the (acceptor or donor, preferably acceptor) chromophore may be inserted at a position corresponding to the position between amino acid positions 522 and 531 or (preferably directly) C-terminally of amino acid position 531 of SEQ ID NO: 1 (e.g., between amino acid positions 522 and 523 of SEQ ID NO: 1), at a position corresponding to the position between amino acid positions 511 and 520 or (preferably directly) C-terminally of amino acid position 520 of SEQ ID NO: 2 (e.g., between amino acid positions 511 and 512 of SEQ ID NO: 2), at a position corresponding to the position between amino acid positions 592 and 610 or (preferably directly) C-terminally of amino acid position 610 of SEQ ID NO: 3 (e.g., between amino acid positions 592 and 593 of SEQ ID NO: 3), at a position corresponding to the position between amino acid positions 547 and 553 or (preferably directly) C-terminally of amino acid position 553 of SEQ ID NO: 4 (e.g., between amino acid positions 547 and 548 of SEQ ID NO: 4), at a position corresponding to the position between amino acid positions 509 and 518 or (preferably directly) C-terminally of amino acid position 518 of SEQ ID NO: 5 (e.g., between amino acid positions 509 and 510 of SEQ ID NO: 5), at a position corresponding to the position between amino acid positions 474 and 494 or (preferably directly) C-terminally of amino acid position 494 of SEQ ID NO: 6 (e.g., between amino acid positions 474 and 475 of SEQ ID NO: 6), at a position corresponding to the position between amino acid positions 471 and 491 or (preferably directly) C-terminally of amino acid position 491 of SEQ ID NO: 7 (e.g., between amino acid positions 471 and 472 of SEQ ID NO: 7), at a position corresponding to the position between amino acid positions 475 and 541 or (preferably directly) C-terminally of amino acid position 541 of SEQ ID NO: 8 (e.g., between amino acid positions 475 and 476 of SEQ ID NO: 8), at a position corresponding to the position between amino acid positions 475 and 488 or (preferably directly) C-terminally of amino acid position 488 of SEQ ID NO: 9 (e.g., between amino acid positions 475 and 476 of SEQ ID NO: 9), at a position corresponding to the position between amino acid positions 521 and 524 or (preferably directly) C-terminally of amino acid position 524 of SEQ ID NO: 10 (e.g., between amino acid positions 521 and 522 of SEQ ID NO: 10), or (preferably directly) C-terminally of a position corresponding to the position of amino acid position 507 of SEQ ID NO: 11. In a specific embodiment of the present invention, the (acceptor or donor, preferably acceptor) chromophore may be inserted at a position corresponding to the position between amino acid positions 522 and 523 of SEQ ID NO: 1 or at a position corresponding to the position between amino acid positions 511 and 512 of SEQ ID NO: 2.

**Table 1:** Sequences of C(D)PKs and indication of protein kinase domains (PKD), pseudosubstrate segment (PS), and a calmodulin-like domain (CLD), said CLD comprising 4 EF-hand motifs EF1, EF2, EF3 and EF4. SEQ ID NOs. refer to full CDPK polypeptide sequences including N-terminal variable domain (VD) and short variable regions between PKD and PS. C-Terminus: Variable C-terminal region after 4th EF hand motif of CLD.

| C(D)PK | SEQ ID NO: | PKD | PS | CLD | C-Terminus |
|---|---|---|---|---|---|
| *Arabidopsis thaliana* CDPK21 | 1 | 80-338 | 343-373 | 374-522 | 523-531 |

(continued)

| C(D)PK | SEQ ID NO: | PKD | PS | CLD | C-Terminus |
|---|---|---|---|---|---|
| *Arabidopsis thaliana* CDPK23 | 2 | 69-327 | 332-362 | 363-511 | 512-520 |
| *Arabidopsis thaliana* CDPK1 | 3 | 150-408 | 414-444 | 445-592 | 593-610 |
| *Solanum lycopersicum* SlCPK1 | 4 | 105-363 | 368-398 | 399-547 | 548-553 |
| *Oryza sativa* OsaCPK1 | 5 | 66-324 | 330-360 | 361-509 | 510-518 |
| *Selaginella moellendorffii* Smo99178 | 6 | 32-290 | 296-326 | 327-474 | 475-494 |
| *Physcomitrella patens* Ppa1s49_208V6 PpCPK12 | 7 | 29-287 | 293-323 | 324-471 | 472-491 |
| *Chlamydomonas reinhardtii* Cre33.g782750 | 8 | 34-292 | 298-328 | 329-475 | 476-541 |
| *Volvox carteri* Vca20014333m | 9 | 34-292 | 298-328 | 329-475 | 476-488 |
| *Plasmodium falciparum* PfaCDPK1 | 10 | 56-325 | 334-364 | 365-521 | 522-524 |
| *Toxoplasma gondii* TgCDPK1 | 11 | 51-308 | 317-347 | 348-507 | n/a |

In accordance with the present invention, the donor chromophore and the acceptor chromophore to be inserted into the respective sites of the C(D)PK represent a Förster Resonance Energy Transfer (FRET) pair. The principle of FRET is described in, e.g., Helms, "Fluorescence Resonance Energy Transfer", Principles of Computational Cell Biology, Weinheim: Wiley-VCH, pp. 202, ISBN 978-3-527-31555-0, and also suitable chromophore pairs are known in the art as described in, e.g., Bajar et al., Sensors (2016), 16: 1488, doi: 10.3390/s16091488. In principle, a donor chromophore transfers energy in its excited state to an acceptor chromophore through non-radiative dipole-dipole coupling. FRET may then be measured upon special vicinity of the two chromophores when light is emitted.

In one embodiment of the present invention, the chromophores are fluorescent proteins (FPs), non-naturally occurring amino acids, small organic dyes, or quantum dots (QDs). FPs are known in the art and their employment in FRET assays is described in, e.g., Bajar, loc.cit. The principle, preparation, introduction and employment of non-naturally occurring amino acids in FRET assays is known the art as well and described in, e.g., Ko et al., RSC Advances (2016), 6(82): 78661-78668. In preferred embodiments of the present invention, the chromophores to be employed as described and provided herein are FPs and/or non-naturally occurring amino acids. In a specific embodiment of the present invention, the chromophores to be employed as described and provided herein are FPs.

As known in the art and as also in accordance with the present invention, some chromophores (e.g., FPs) can serve both as donor chromophore and as acceptor chromophore in a FRET assay, dependent on the respective other chromophore of the FRET pair; cf. also Bajar, loc. cit. The selection of suitable FRET pairs depends on, e.g., the apparatus used for measuring the light (not all wavelength can be measured equally by different apparatuses) and the expected radius of interaction (the radius of interaction is smaller than the wavelength of the emitted light). Table 2 provides general examples of suitable FRET chromophore pairs employable in accordance with the present invention in relation to colors.

**Table 2:** Suitable FRET chromophore pairs (colors). (e)CFP: (enhanced) Cyan Fluorescent Protein; YFP: Yellow Fluorescent Protein; GFP: Green Fluorescent Protein; RFP: Red Fluorescent Protein.

| Donor chromophore | Acceptor chromophore |
| --- | --- |
| (e)CFP | YFP or GFP (preferably YFP) |
| GFP or YFP (preferably GFP) | RFP |
| RFP | Near-infrared FP |
| Non-naturally occurring amino acid (e.g., CouA) | YFP or RFP (preferably YFP) |

Specific examples of CFPs, GFPs, YFPs and RFPs which can be used in accordance with the present invention as FRET pair chromophores are known in the art (cf., e.g., Bajar, loc. cit.) and also described herein.

For example, in one embodiment of the present invention, a CFP chromophore may be selected from the group consisting of mTurquoise (mT), (e)CFP, Aquamarine, mTurquoise2 (mT2), mCerulean3, mTFP1 and LUMP. In a specific embodiment of the present invention, CFP is mT or (e)CFP. In a further specific embodiment, the CFP (e.g., mT or eCFP) is used as donor chromophore for a YFP or GFP (preferably YFP) acceptor chromophore, e.g., cpVenus173.

For example, in one embodiment of the present invention, a YFP chromophore may be selected from the group consisting of cpVenus173, eYFP, mVenus, Venus, mCitrine, sEYFP and YPet. In a specific embodiment of the present invention, YFP is cpVenus173. In a further specific embodiment, the YFP (e.g., cpVenus173) is used as acceptor chromophore for a CFP donor chromophore, e.g., mT or (e)CFP.

For example, in one embodiment of the present invention, a GFP chromophore may be selected from the group consisting of eGFP, NowGFP, Clover, mClover3 and mNeonGreen. In a further specific embodiment, the GFP is used as donor chromophore for a RFP acceptor chromophore.

For example, in one embodiment of the present invention, a RFP chromophore may be selected from the group consisting of mRuby2, mRuby3, mRFP, nKOk, and mCherry. In a further specific embodiment, the RFP is used as acceptor chromophore for a GFP or YFP (preferably GFP) donor chromophore.

In certain embodiments of the present invention,

(a) the donor chromophore to be inserted into the C(D)PK as described and provided herein may be a cyan FP (CFP) and the acceptor chromophore may be a yellow FP (YFP),
(b) the donor chromophore to be inserted into the C(D)PK as described and provided herein may be a green FP (GFP) or yellow FP (YFP), the acceptor chromophore may be a red FP (RFP),
(c) the donor chromophore to be inserted into the C(D)PK as described and provided herein may be a cyan FP (CFP), the acceptor chromophore may be a green FP (GFP),
(d) the donor chromophore to be inserted into the C(D)PK as described and provided herein may be a red FP (RFP), and the acceptor chromophore may be a near-infrared FP, or
(e) the donor chromophore to be inserted into the C(D)PK as described and provided herein may be a non-naturally occurring amino acid (e.g., CouA; cf. Ko et al., loc. cit.) and the acceptor chromophore may be an FP (for example YFP or RFP, preferably YFP).

For example, in accordance with the present invention, the donor chromophore to be inserted into the C(D)PK as described and provided herein may be a cyan FP (CFP) and the acceptor chromophore may be a yellow FP (YFP), or the donor chromophore to be inserted into the C(D)PK as described and provided herein is a green FP (GFP), the acceptor chromophore is a red FP (RFP). In a specific embodiment of the present invention, the donor chromophore to be inserted into the C(D)PK as described and provided herein is a cyan FP (CFP) and the acceptor chromophore is a yellow FP (YFP).

Specific examples for chromophore pairs according to the color (CFP, YFP, GFP, RFP) which can be used in accordance with the present invention are provided in Table 3. Further suitable pairs are known in the art and described in, e.g., Bajar, loc. cit.

**Table 3:** Specific examples for FP chromophore pairs in accordance with the present invention. Chromophore pairs are indicated in the respective lines in the 2nd and 3rd column of the table. Preferred pairs in bold, particularly preferred pairs also underlined.

| Donor chromophore | | Acceptor chromophore | |
|---|---|---|---|
| Chromophore color class | Chromophore | Chromophore | Chromophore color class |
| CFP | **mTurquoise (mT)** | **cpVenus173** | YFP |
| | **(e)CFP** | **cpVenus173** | |
| | **(e)CFP** | **eYFP** | |
| | **mTurquoise2 (mT2)** | **mVenus** | |
| | LUMP | Venus | |
| GFP | **eGFP** | **mCherry** | RFP |
| | Clover | mRuby2 | |
| | **mClover3** | **mRuby3** | |
| | **mNeonGreen** | **mRuby3** | |
| YFP | cpVenus173 | mCherry | |
| | cpVenus173 | mRFP | |
| | cpVenus173 | nKOk | |

Accordingly, in one embodiment of the present invention,

(i) the donor chromophore may be selected from the group consisting of mTurquoise (mT), eCFP, Aquamarine, mTurquoise2 (mT2), mCerulean3, mTFP1 and LUMP, and the acceptor chromophore may be selected from the group consisting of cpVenus173, eYFP, mVenus, Venus, mCitrine, sEYFP and YPet, or
(ii) the donor chromophore may be selected from the group consisting of cpVenus173, eGFP, NowGFP, Clover, mClover3 and mNeonGreen, and

the acceptor chromophore may be selected from the group consisting of mRuby2, mRuby3, mRFP, nKOk, and mCherry.

As described herein, the donor chromophore is inserted into the C(D)PK between the PKD and the PS. As described herein, in context with the present invention, the donor chromophore may be inserted directly at the C-Terminus of the PKD, directly at the N-Terminus of the PS, or between the C-Terminus of the PKD and the N-Terminus of the PS; cf. also Table 1.

As described herein, the acceptor chromophore is inserted into the C(D)PK between C-terminally of EF4 (i.e. C-terminally of the 4th EF hand motif of the CLD). As described herein, in context with the present invention, the acceptor chromophore may be inserted directly at the C-Terminus of EF4, at the C-Terminus of the CDPK polypeptide, or between the C-Terminus of EF4 and the C-Terminus of the CDPK polypeptide; cf. also Table 1 herein ("C-Terminus").

In specific embodiments of the present invention, the amino acid sequence comprising the PKD, the PS and the CLD of the C(D)PK is at least 37%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% identical to the amino acid sequence 80-522 of SEQ ID NO: 1, 69-511 of SEQ ID NO: 2, 150-592 of SEQ ID NO: 3, 105-547 of SEQ ID NO: 4, 66-509 of SEQ ID NO: 5, 32-474 of SEQ ID NO: 6, 29-471 of SEQ ID NO: 7,34-475 of SEQ ID NO: 8, 34-475 of SEQ ID NO: 9, 56-521 of SEQ ID NO: 10, or 51-507 of SEQ ID NO: 11. In a further specific embodiment, the amino acid sequence comprising the PKD, the PS and the CLD of the C(D)PK is at least 37%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% identical to the amino acid sequence 80-522 of SEQ ID NO: 1, or 69-511 of SEQ ID NO: 2.

The level of identity between two or more sequences (e.g., nucleic acid sequences or amino acid sequences) can be easily determined by methods known in the art, e.g., by BLAST analysis. Generally, in context with the present invention, if two sequences (e.g., polynucleotide sequences or amino acid sequences) to be compared by, e.g., sequence comparisons differ in identity, then the term "identity" may refer to the shorter sequence and that part of the longer sequence that matches said shorter sequence. Therefore, when the sequences which are compared do not have the same length, the degree of identity may preferably either refer to the percentage of nucleotide residues in the shorter sequence which are identical to nucleotide residues in the longer sequence or to the percentage of nucleotides in the longer sequence which are identical to nucleotide sequence in the shorter sequence. In this context, the skilled person is readily in the position to determine that part of a longer sequence that matches the shorter sequence. Furthermore, as used herein, identity levels of nucleic acid sequences or amino acid sequences may refer to the entire length of the respective sequence and is preferably assessed pair-wise, wherein each gap is to be counted as one mismatch. These definitions for sequence comparisons (e.g., establishment of "identity" values) are to be applied for all sequences described and disclosed herein.

Moreover, the term "identity" as used herein means that there is a functional and/or structural equivalence between the corresponding sequences. Nucleic acid/amino acid sequences having the given identity levels to the herein-described particular nucleic acid/amino acid sequences may represent derivatives/variants of these sequences which, preferably, have the same biological function. They may be either naturally occurring variations, for instance sequences from other varieties, species, etc., or mutations, and said mutations may have formed naturally or may have been produced by deliberate mutagenesis. Furthermore, the variations may be synthetically produced sequences. The variants may be naturally occurring variants or synthetically produced variants or variants produced by recombinant DNA techniques. Deviations from the above-described nucleic acid sequences may have been produced, e.g., by deletion, substitution, addition, insertion and/or recombination. The term "addition" refers to adding at least one nucleic acid residue/amino acid to the end of the given sequence, whereas "insertion" refers to inserting at least one nucleic acid residue/amino acid within a given sequence. The term "deletion" refers to deleting or removal of at least one nucleic acid residue or amino acid residue in a given sequence. The term "substitution" refers to the replacement of at least one nucleic acid residue/amino acid residue in a given sequence. Again, these definitions as used here apply, *mutatis mutandis*, for all sequences provided and described herein.

In specific embodiments of the present invention, the amino acid sequence comprising the PKD, the PS and the CLD of the C(D)PK is at least 37%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% similar to the amino acid sequence 80-522 of SEQ ID NO: 1, 69-511 of SEQ ID NO: 2, 150-592 of SEQ ID NO: 3, 105-547 of SEQ ID NO: 4, 66-509 of SEQ ID NO: 5, 32-474 of SEQ ID NO: 6, 29-471 of SEQ ID NO: 7, 34-475 of SEQ ID NO: 8, 34-475 of SEQ ID NO: 9, 56-521 of SEQ ID NO: 10, or 51-507 of SEQ ID NO: 11. In a further specific embodiment, the amino acid sequence comprising the PKD, the PS and the CLD of the C(D)PK is at least 37%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% similar to the amino acid sequence 80-522 of SEQ ID NO: 1, or 69-511 of SEQ ID NO: 2.

In context with the present invention, the term "similar" with respect to comparisons of amino acid sequences mean that no substitution or only conservative or highly conservative amino acid substitutions compared to a given amino acid sequence are considered "similar". Conservative amino acid substitutions are known in the art, and include amino acid substitutions in which one amino acid having certain physical and/or chemical properties is exchanged for another amino acid that has the same chemical or physical properties. For instance, the conservative amino acid substitution can be in an acidic amino acid substituted for another acidic amino acid, an amino acid with a nonpolar side chain substituted for another amino acid with a nonpolar side chain, a basic amino acid substituted for another basic amino acid, an amino acid with a polar side chain substituted for another amino acid with a polar side chain, etc. that may be made, for instance, on the basis of similarity in polarity, charge, solubility, hydrophobicity, hydrophilicity, and/or the amphipathic nature of the residues involved. As used herein, "silent" mutations mean base substitutions within a nucleic acid sequence which do not change the amino acid sequence encoded by the nucleic acid sequence. "Conservative" substitutions mean substitutions as listed as "Exemplary Substitutions" in Table I below. "Highly conservative" substitutions as used herein mean substitutions as shown under the heading "Preferred Substitutions" in Table I below. The term "addition" as used herein refers to adding at least one nucleic acid residue/amino acid to the end of the given sequence, whereas "insertion" refers to inserting at least one nucleic acid residue/amino acid within a given sequence. The term "deletion" refers to deleting or removal of at least one nucleic acid residue or amino acid residue in a given sequence. The term "substitution" refers to the replacement of at least one nucleic acid residue/amino acid residue in a given sequence. These definitions as used here apply, *mutatis mutandis*, for all sequences provided and described herein.

The term "position" when used in accordance with the present invention means the position of an amino acid within an amino acid sequence depicted herein. The term "corresponding" in this context also includes that a position is not only determined by the number of the preceding nucleotides/amino acids. It also includes that a C(D)PK may be similar to a given C(D)PK as depicted in any of SEQ ID NOs. 1 to 11 herein to a level of similarity defined herein, and the insertion positions of the donor and the acceptor chromophores as described herein into the similar C(D)PK correspond to the respective positions of the C(D)PKs represented by SEQ ID NOs. 1 to 11.

TABLE I Amino Acid Substitutions

| Original | Exemplary Substitutions | Preferred Substitutions |
|---|---|---|
| Ala (A) | val; leu; ile | Val |
| Arg (R) | lys; gin; asn | lys |
| Asn (N) | gin; his; asp, lys; arg | gin |
| Asp (D) | glu; asn | glu |
| Cys (C) | ser; ala | ser |
| Gln (Q) | asn; glu | asn |
| Glu (E) | asp; gln | asp |
| Gly (G) | ala | ala |
| His (H) | asn; gln; lys; arg | arg |
| Ile (1) | leu; val; met; ala; phe; | leu |
| Leu (L) | norleucine; ile; val; met; ala; | ile |
| Lys (K) | arg; gin; asn | arg |
| Met (M) | leu; phe; ile | leu |
| Phe (F) | leu; val; ile; ala; tyr | tyr |
| Pro (P) | ala | ala |
| Ser (S) | thr | thr |
| Thr (T) | ser | ser |
| Trp (W) | tyr; phe | tyr |
| Tyr (Y) | trp; phe; thr; ser | Phe |
| Val (V) | ile; leu; met; phe; ala; | leu |

Unless otherwise specified herein, the term "polypeptide" as used herein is to be construed interchangeably with the terms "amino acid sequence", "peptide chain" or "protein".

In specific embodiments of the present invention, the amino acid sequence of the C(D)PK comprising the PKD is at least 44%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% identical to the amino acid sequence 80 to 338 of SEQ ID NO: 1, 69-327 of SEQ ID NO: 2, 150-408 of SEQ ID NO: 3, 105-363 of SEQ ID NO: 4, 66-324 of SEQ ID NO: 5, 32-290 of SEQ ID NO: 6, 29-287 of SEQ ID NO: 7, 34-292 of SEQ ID NO: 8, 34-292 of SEQ ID NO: 9, 56-325 of SEQ ID NO: 10, or 51-308 of SEQ ID NO: 11. In a further specific embodiment of the present invention, the amino acid sequence of the C(D)PK comprising the PKD is at least 44%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% identical to the amino acid sequence 80 to 338 of SEQ ID NO: 1, or 69-327 of SEQ ID NO: 2.

In specific embodiments of the present invention, the amino acid sequence of the C(D)PK comprising the PKD is at least 44%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% similar to the amino acid sequence 80 to 338 of SEQ ID NO: 1, 69-327 of SEQ ID NO: 2, 150-408 of SEQ ID NO: 3, 105-363 of SEQ ID NO: 4, 66-324 of SEQ ID NO: 5, 32-290 of SEQ ID NO: 6, 29-287 of SEQ ID NO: 7, 34-292 of SEQ ID NO: 8, 34-292 of SEQ ID NO: 9, 56-325 of SEQ ID NO: 10, or 51-308 of SEQ ID NO: 11. In a further specific embodiment of the present invention, the amino acid sequence of the C(D)PK comprising the PKD is at least 44%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% similar to the amino acid sequence 80 to 338 of SEQ ID NO: 1, or 69-327 of SEQ ID NO: 2.

In specific embodiments of the present invention, the amino acid sequence of the C(D)PK comprising the PS is at least 23%, 25%, 30%, 35%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% identical to the amino acid sequence 343 to 373 of SEQ ID NO: 1, 332-362 of SEQ ID NO: 2, 414-444 of SEQ ID NO: 3, 368-398 of SEQ ID NO: 4, 330-360 of SEQ ID NO: 5, 296-326 of SEQ ID NO: 6, 293-323 of SEQ ID NO: 7, 298-328 of SEQ ID NO: 8, 298-328 of SEQ ID NO:

9, 334-364 of SEQ ID NO: 10, or 317-347 of SEQ ID NO: 11. In a further specific embodiment of the present invention, the amino acid sequence of the C(D)PK comprising the PS is at least 23%, 25%, 30%, 35%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% identical to the amino acid sequence 343 to 373 of SEQ ID NO: 1, or 332-362 of SEQ ID NO: 2.

In specific embodiments of the present invention, the amino acid sequence of the C(D)PK comprising the PS is at least 23%, 25%, 30%, 35%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% similar to the amino acid sequence 343 to 373 of SEQ ID NO: 1, 332-362 of SEQ ID NO: 2, 414-444 of SEQ ID NO: 3, 368-398 of SEQ ID NO: 4, 330-360 of SEQ ID NO: 5, 296-326 of SEQ ID NO: 6, 293-323 of SEQ ID NO: 7, 298-328 of SEQ ID NO: 8, 298-328 of SEQ ID NO: 9, 334-364 of SEQ ID NO: 10, or 317-347 of SEQ ID NO: 11. In a further specific embodiment of the present invention, the amino acid sequence of the C(D)PK comprising the PS is at least 23%, 25%, 30%, 35%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% similar to the amino acid sequence 343 to 373 of SEQ ID NO: 1, or 332-362 of SEQ ID NO: 2.

In specific embodiments of the present invention, the amino acid sequence of the C(D)PK comprising the CLD is at least 27%, 30%, 35%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% identical to the amino acid sequence 374 to 522 of SEQ ID NO: 1, 363-511 of SEQ ID NO: 2, 445-592 of SEQ ID NO: 3, 399-547 of SEQ ID NO: 4, 361-509 of SEQ ID NO: 5, 327-474 of SEQ ID NO: 6, 324-471 of SEQ ID NO: 7, 329-475 of SEQ ID NO: 8, 329-475 of SEQ ID NO: 9, 365-521 of SEQ ID NO: 10, or 348-507 of SEQ ID NO: 11. In a further specific embodiment of the present invention, the amino acid sequence of the C(D)PK comprising the CLD is at least 27%, 30%, 35%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% identical to the amino acid sequence 374 to 522 of SEQ ID NO: 1, or 363-511 of SEQ ID NO: 2.

In specific embodiments of the present invention, the amino acid sequence of the C(D)PK comprising the CLD is at least 27%, 30%, 35%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% similar to the amino acid sequence 374 to 522 of SEQ ID NO: 1, 363-511 of SEQ ID NO: 2, 445-592 of SEQ ID NO: 3, 399-547 of SEQ ID NO: 4, 361-509 of SEQ ID NO: 5, 327-474 of SEQ ID NO: 6, 324-471 of SEQ ID NO: 7, 329-475 of SEQ ID NO: 8, 329-475 of SEQ ID NO: 9, 365-521 of SEQ ID NO: 10, or 348-507 of SEQ ID NO: 11. In a further specific embodiment of the present invention, the amino acid sequence of the C(D)PK comprising the CLD is at least 27%, 30%, 35%, 40%, 45%, 50%, 55%, 60%, 65%, 70%, 75%, 80%, 82%, 83%, 84%, 85%, 86%, 87%, 88%, 89%, 90%, 91%, 92%, 93%, 94%, 95%, 96%, 97%, 98% 99%, or 99.5% similar to the amino acid sequence 374 to 522 of SEQ ID NO: 1, or 363-511 of SEQ ID NO: 2.

In a further embodiment of the present invention, the C(D)PK polypeptide comprises an amino acid sequence which is at least 70%, 75%, 80%, 82%, 83%, 84%, 85%, 86%, 87%, 88%, 89%, 90%, 91%, 92%, 93%, 94%, 95%, 96%, 97%, 98% 99%, or 99.5% identical to SEQ ID NO: 1 without taking into account the inserted chromophores and any linkers, if applicable.

In a further embodiment of the present invention, the C(D)PK polypeptide comprises an amino acid sequence which is at least 70%, 75%, 80%, 82%, 83%, 84%, 85%, 86%, 87%, 88%, 89%, 90%, 91%, 92%, 93%, 94%, 95%, 96%, 97%, 98% 99%, or 99.5% similar to SEQ ID NO: 1 without taking into account the inserted chromophores and any linkers, if applicable.

The present invention also relates to polynucleotides encoding the C(D)PK polypeptide as described and provided herein.

Generally, as used herein, the terms "polynucleotide" and "nucleic acid" or "nucleic acid molecule" are to be construed synonymously. Generally, nucleic acid molecules may comprise *inter alia* DNA molecules, RNA molecules, oligonucleotide thiophosphates, substituted ribo-oligonucleotides or PNA molecules. Furthermore, the term "nucleic acid molecule" may refer to DNA or RNA or hybrids thereof or any modification thereof that is known in the art (see, e.g., US 5525711, US 471 1955, US 5792608 or EP 302175 for examples of modifications). The polynucleotide sequence may be single- or doublestranded, linear or circular, natural or synthetic, and without any size limitation. For instance, the polynucleotide sequence may be genomic DNA, cDNA, mitochondrial DNA, mRNA, antisense RNA, ribozymal RNA or a DNA encoding such RNAs or chimeroplasts (Gamper, Nucleic Acids Research, 2000, 28, 4332 - 4339). Said polynucleotide sequence may be in the form of a vector, plasmid or of viral DNA or RNA. Also described herein are nucleic acid molecules which are complementary to the nucleic acid molecules described above and nucleic acid molecules which are able to hybridize to nucleic acid molecules described herein. A nucleic acid molecule described herein may also be a fragment of the nucleic acid molecules in context of the present invention. Particularly, such a fragment is a functional fragment. Examples for such functional fragments are nucleic acid molecules which can serve as primers.

The present invention further relates to vectors comprising a polynucleotide encoding the C(D)PK polypeptide as described and provided herein.

The term "vector" as used herein particularly refers to plasmids, cosmids, viruses, bacteriophages and other vectors commonly used in genetic engineering. In one embodiment of the present invention, the vectors are suitable for the transformation, transduction and/or transfection of host cells as described herein, e.g., prokaryotic cells (e.g., (eu)bacteria, archaea), eukaryotic cells (e.g., mammalian cells, insect cells, fungal cells, yeast), and the like. Examples of bacterial host cells in context with the present invention comprise Gram negative and Gram positive cells. Specific examples for suitable host cells may comprise inter alia *E. coli* (e.g., BL21 or Rosetta), *Toxoplasma gondi*, *Plasmodium falciparum*, *Arabidopsis thaliana* cells, or any cell (preferably plant cell) naturally comprising the C(D)PK from which the polypeptide of the present invention is derived by inserting the chromophores as described and provided herein (cf. also list of C(D)PKs in Table 1 with their respective natural origin organism). In one embodiment of the present invention, said vectors are suitable for stable transformation in the host cells, for example to express the C(D)PK polypeptide as described and provided herein.

Accordingly, in one aspect of the invention, the vector as provided is an expression vector. Generally, expression vectors have been widely described in the literature. As a rule, they may not only contain a selection marker gene and a replication-origin ensuring replication in the host selected, but also a promoter, and in most cases a termination signal for transcription. Between the promoter and the termination signal there is preferably at least one restriction site or a polylinker which enables the insertion of a nucleic acid sequence/molecule desired to be expressed. It is to be understood that when the vector provided herein is generated by taking advantage of an expression vector known in the prior art that already comprises a promoter suitable to be employed in context of this invention, for example expression of a C(D)PK polypeptide as described herein. The nucleic acid construct is preferably inserted into that vector in a manner the resulting vector comprises only one promoter suitable to be employed in context of this invention. The skilled person knows how such insertion can be put into practice. For example, the promoter can be excised either from the nucleic acid construct or from the expression vector prior to ligation. In one embodiment of the present invention, the vector is able to integrate into the host cell genome. The vector may be any vector suitable for the respective host cell, preferably an expression vector. The vector may comprise the polynucleotide encoding a C(D)PK polypeptide as described and also provided herein. Generally suitable vectors in this context comprise inter alia those with ubiquitin promoters or 35S promoters. Non-limiting examples of the vector of the present invention may comprise pET30a-c (Novagen), pRSET vectoren (Thermo Fisher), pGEX-6P/4T/5X (Sigma Aldrich), or pXCS-HA-Strep (cf. Franz et al., Mol Plant (2011), 4(1): 83-96), each comprising the polynucleotide in context of the present invention. Non-limiting examples for suitable promoters comprise inter alia pGC1 (Stomata), pSUC2 (Phloem), pLOX6 (Xylem), or pCPK21.

The present invention further relates to host cells comprising the C(D)PK polypeptide, the polynucleotide encoding the C(D)PK polypeptide, and/or the vector comprising the polynucleotide encoding the C(D)PK polypeptide as described and provided herein.

The host cell of the present invention may generally be any host cell, preferably being capable of stably expressing a polynucleotide encoding the C(D)PK polypeptide as described and provided herein. Such host cells may comprise, inter alia, e.g., prokaryotic cells (e.g., (eu)bacteria, archaea), eukaryotic cells (e.g., mammalian cells (e.g., HEK cells), insect cells) fungal cells, yeast, or entire organisms (e.g., *Arabidopsis thaliana)* and the like. In one embodiment of the present invention, the host cell is not archaea. Examples of bacterial host cells in context with the present invention comprise Gram negative and Gram positive cells. Specific examples for suitable host cells in context with the present invention may comprise inter alia *E. coli* (e.g., BL21 or Rosetta), *Toxoplasma gondi*, *Plasmodium falciparum*, *Arabidopsis thaliana* cells, or any cell (preferably plant cell) naturally comprising the C(D)PK from which the polypeptide of the present invention is derived by inserting the chromophores as described and provided herein (cf. also list of C(D)PKs in Table 1 with their respective natural origin organism).

The present invention further relates to methods for measuring conformational change or activation status of a CDPK polypeptide, comprising the following steps:

(1) cultivating the host cell as described and provided herein comprising the C(D)PK polypeptide of the present invention under suitable conditions,
(2) optionally adding calcium and/ or applying stress treatment (such as, e.g., phytohormone treatment (e.g., abscisic acid), abiotic stress treatment (e.g., cold, salt), or biotic stress treatment (e.g., flagellin, chitin)) to the host cell, and
(3) measuring FRET occurrence.

For the avoidance of doubt, such cultivation and measuring is possible both, *in vivo* and *in vitro.* For plant cells as host cells, *in vivo* may be preferred, while for animal or human cells, *in vitro* is preferred.

The present invention further relates to use of a C(D)PK polypeptide comprising chromophores as described and provided in accordance with the present invention, or of the the polynucleotide encoding the C(D)PK polypeptide, and/or the vector comprising the polynucleotide encoding the C(D)PK polypeptide, or of the host cell as described and provided herein, for measuring conformational change or activation status of a CDPK polypeptide.

The embodiments, which characterize the present invention, are described herein, shown in the Figures, illustrated in the Examples, and reflected in the claims.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or", wherever used herein, includes the meaning of "and", "or" and "all or any other combination of the elements connected by said term".

The term "about" or "approximately" as used herein means within 20%, preferably within 10%, more preferably within 5%, and most preferably within 3% of a given value or range.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

Unless specifically stated otherwise, in each instance herein any of the terms "comprising", "consisting essentially of" and "consisting of" may be replaced with either of the other two terms. For example, where a given feature, compound or range is indicted as "comprised by" a respective broader term, such broader term may also "consist of" such feature, compound or range.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

The Figures show:

**Figure** 1     Kinase activity of CPK21 and CPK23 plotted against $Ca^{2+}$-concentrations ($R^2_{CPK21}$ = 0.91, $R^2_{CPK23}$ = 0.76). Activity is expressed as percentage of full $Ca^{2+}$-saturation (means $\pm$ SEM n= 2-3).

**Figure** 2     Comparison of FRET-recorded conformational change of CPK21D204A and CPK23D193A plotted against $[Ca^{2+}]$ ($R^2_{CPK21D204A}$ = 0.98, $R^2_{CPK23D193A}$ = 0.69). FRET efficiency change is given as percentage of increase in ratio over the baseline FRET signal (means $\pm$ SEM n = 3-6).

**Figure** 3     Mean $\pm$ SEM of half-maximal kinase activity (K50) or half-maximal effective concentration (EC50) of conformational change in n independent experiments. The Hillslope was fitted as shared value for all data sets of the same enzyme.

**Figure** 4     Kinase activity of CPK21 PS variants carrying amino acid substitution (CPK21I373S, CPK21I373D) plotted against $[Ca^{2+}]$ ($R^2_{CPK21}$ = 0.85, $R^2_{CPK21I373S}$ = 0.97, $R^2_{CPK21I373D}$ = 0.87). Activity is expressed as percentage of full $Ca^{2+}$-saturation (means $\pm$ SEM n= 2-3).

**Figure** 5     FRET-recorded conformational change of CPK21D204A-I373S, CPK21D204A-I373D plotted against $[Ca^{2+}]$ ($R^2_{CPK21D204A}$ = 0.96, $R^2_{CPK21D204A-I3735}$ = 0.99, $R^2_{CPK21D204A-I373D}$ = 0.88). FRET efficiency change is determined as in (Fig. 2) (means $\pm$ SEM n= 4-6).

**Figure** 6     Kinase activity of CPK23 PS variants carrying amino acid substitution (CPK23S362I, CPK23S362D) plotted against $[Ca^{2+}]$ ($R^2_{CPK23}$ = 0.84, $R^2_{CPK23S362I}$ = 0.8). Activity is expressed as percentage of full $Ca^{2+}$-saturation (means $\pm$ SEM n= 2-3). CPK23S362D activity indicated in counts per minute (cpm).

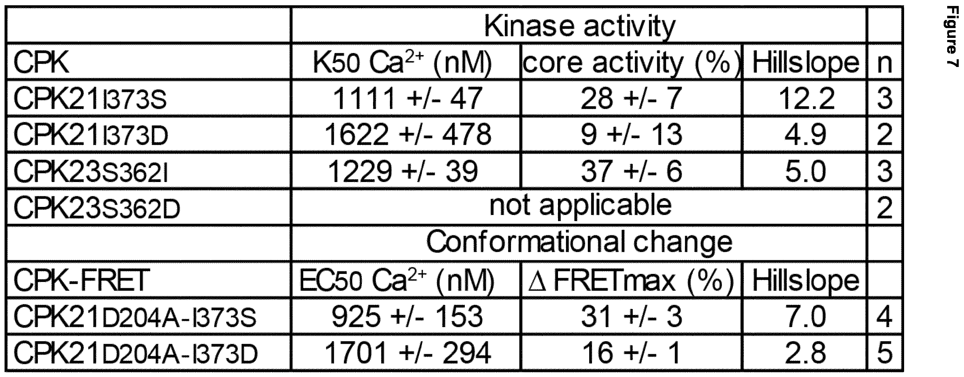

**Figure** 7     Mean $\pm$ SEM for K50 of activity or EC50 of conformational change and shared Hillslope in n independent experiments.

**Figure** 8     Kinase activity of CPK23CLD21 and CPK23S362I-CLD21 plotted against $Ca^{2+}$-concentrations (Kinase activity: $R^2_{CPK21}$ = 0.85, $R^2_{CPK23}$ = 0.89, $R^2_{CPK23CLD21}$ = 0.69, $R^2_{CPK23S362I-CLD21}$ = 0.92). Kinase activity (means $\pm$ SEM n=2-4) is determined as described in Fig. 1.

**Figure 9** FRET efficiency change of CPK23D193A-CLD21 and CPK23D193A-S362I-CLD21 plotted against $Ca^{2+}$-concentrations (Conformational change: $R^2CPK21D204A = 0.99$, $R^2CPK23D193A = 0.81$, $R^2CPK23D193A-CLD21 = 0.91$, $R^2_{CPK2D193A3-S362I-CLD21} = 0.98$). FRET-imaged conformational change (means $\pm$ SEM n= 3-6) are determined as described in Fig. 2.

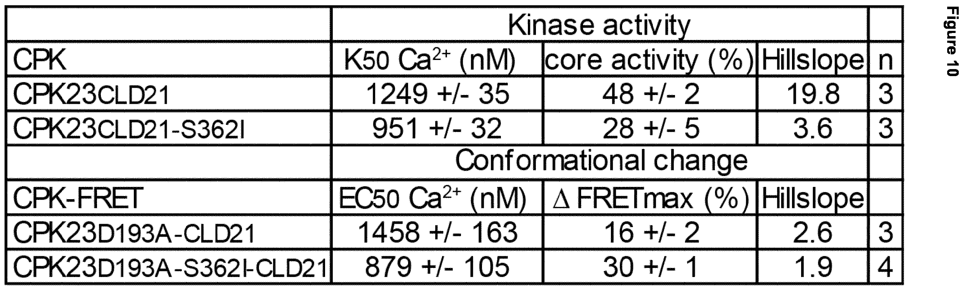

**Figure 10** Mean $\pm$ SEM for K50 of activity or EC50 of conformational change and shared Hillslope in n independent experiments.

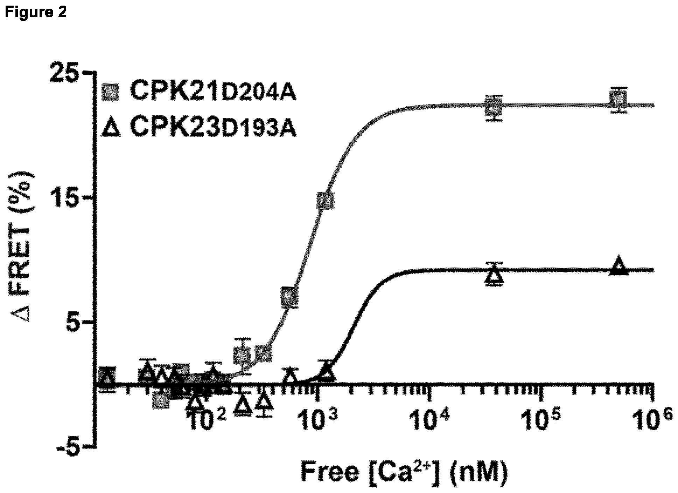

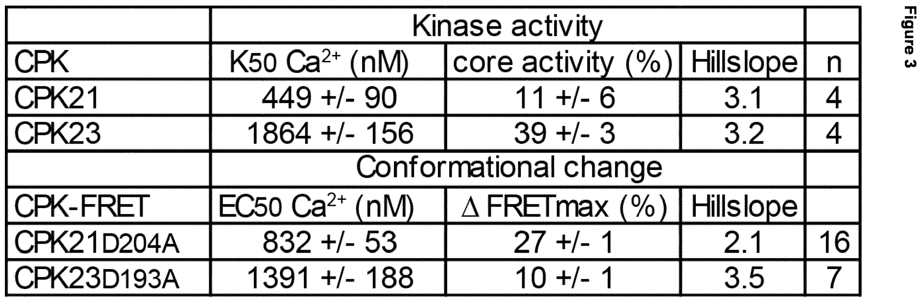

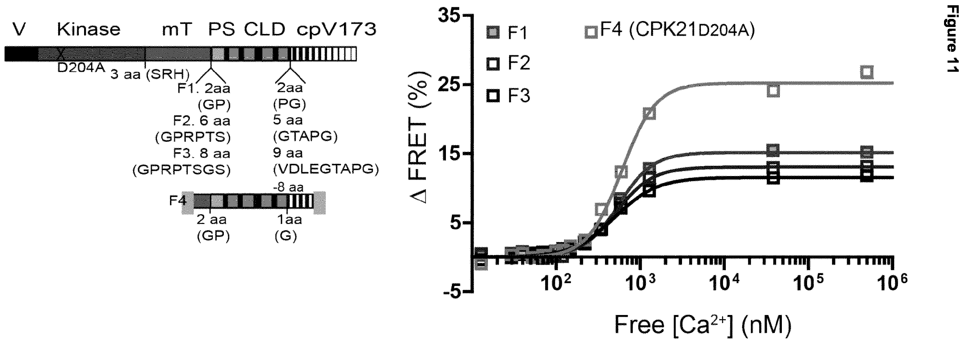

**Figure 11** $Ca^{2+}$-dependent conformational change of CPK21D204A linker variants F1-F4 (right panel). V, variable domain; Kinase, kinase domain; PS, pseudosubstrate segment; CLD, calmodulin-like domain containing four EF-hand motifs (bright boxes); mT, mTurquoise and cpV173, cpVenus173. Numbers indicate the insertion or the deletion of amino acids (aa) with the linker aa sequence shown (left panel). FRET efficiency change is determined as in (Fig. 2) ($R^2_{F1} = 0.99$, $R^2_{F2} = 0.98$, $R^2_{F3} = 0.98$, $R^2_{F4} = 0.99$; means $\pm$ SEM n= 3). Variant F4 shows the highest change in emission ratio and was used as matrix for all further FRET analyses and is denominated as CPK21D204A. The CPK21D204A EC50 value is shown in (Fig. 3).

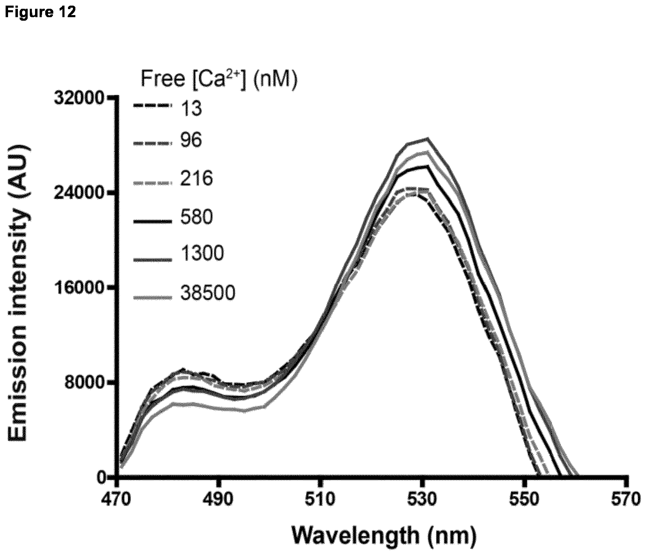

**Figure 12** Emission spectra of CPK21D204A (excited at 435 nm) at the indicated $Ca^{2+}$-concentrations.

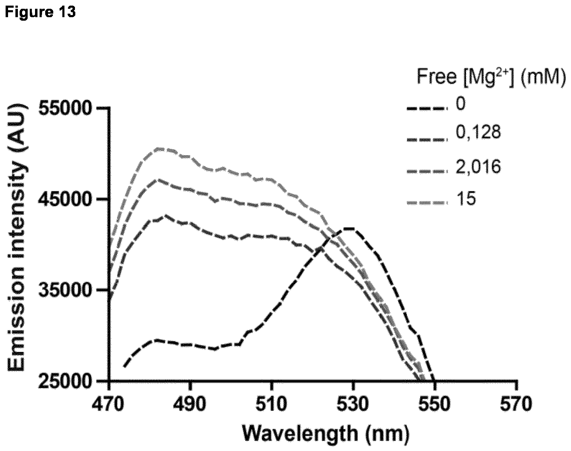

**Figure 13** Emission spectra of CPK21D204A (excited at 435 nm) at the indicated $Mg^{2+}$ concentrations. For $Mg^{2+}$ the experiment was repeated three times with similar results.

**Figure 14** pH-dependency of CPK21D204A FRET efficiency. FRET-recorded conformational change of CPK21D204A (left axis) plotted against pH concentrations in the absence of $Ca^{2+}$ (EGTA, zero free $Ca^{2+}$, means $\pm$ SEM n= 5) and in presence of saturating $[Ca^{2+}]$-concentration (means $\pm$ SEM n= 5). The ratio FRET ($Ca^{2+}$) / FRET (EGTA) is indicated in red (right axis). The experiment was repeated two times with similar results.

**Figure 15** Comparison of FRET-recorded conformational change of CPK21D204A with either mTurquoise (CPK21D204A-FRET) or CFP (CPK21D204A-FRET-CFP) as FRET donor ($R^2_{CPK21D204A-FRET} = 0.94$, $R^2_{CPK21D204A-FRET-CFP} = 0.97$; means $\pm$ SEM n= 4).

**Figure 16** Kinase activity of recombinant CPK21 and CPK21-FRET plotted against $Ca^{2+}$-concentrations ($R^2_{CPK21} = 0.94$; means $\pm$ SEM n= 2-3).

**Figure 17** FRET-recorded conformational change of CPK21-FRET compared with the respective kinase-deficient variants (CPK21D204A-FRET). FRET efficiency change is determined as in (Fig. 2) ($R^2_{CPK21D204A} = 0.96$, $R^2_{CPK21} = 0.9$, $R^2$, means $\pm$ SEM n= 5 - 6).

**Figure 18** FRET-recorded conformational change of CPK23-FRET compared with the respective kinase-deficient variants (CPK23D193A-FRET). FRET efficiency change is determined as in (Fig. 2) ($R^2_{CPK23D193A} = 0.71$, $R^2_{CPK23} = 0.91$; means $\pm$ SEM n= 5 - 6).

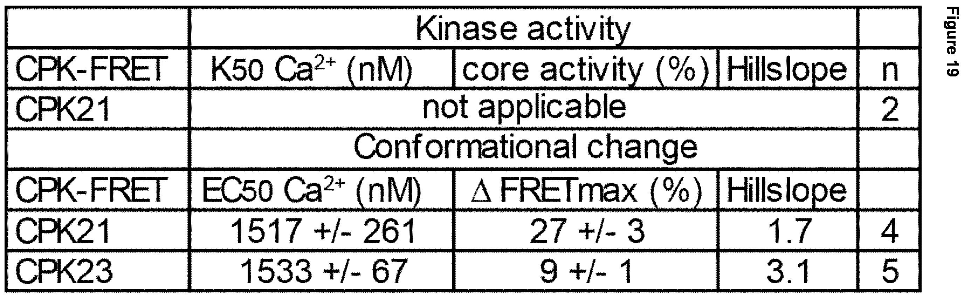

**Figure 19** Mean $\pm$ SEM for K50 of activity or EC50 of conformational change and shared Hillslope in n independent experiments.

**Figure 20** FRET-recorded conformational change of CPK23, CPK23S362I and CPK23S362D plotted against $Ca^{2+}$-concentrations ($R^2_{CPK23} = 0.84$, $R^2_{CPK23S362I} = 0.84$, $R^2_{CPK23S362D} = 0.56$). FRET efficiency change is determined as in (Fig. 3) (means $\pm$ SEM n= 3 - 6).

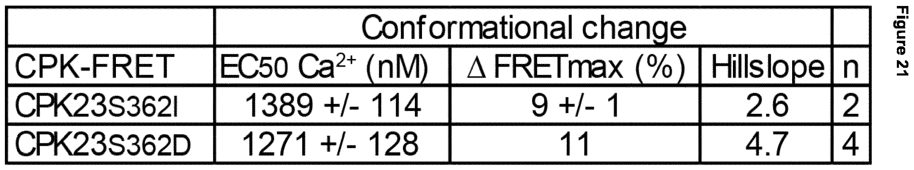

**Figure 21** Mean $\pm$ SEM for K50 of activity or EC50 of conformational change and shared Hillslope in n independent experiments.

**Figure 22** FRET-recorded conformational change of TgCDPK1-FRET plotted against $[Ca^{2+}]$ (*Toxoplasma gondii* CDPK; cf. Table 1, SEQ ID NO: 11 herein). FRET efficiency change is given as percentage of increase in ratio over the baseline FRET signal (means $\pm$ SEM n = 3, EC50 = 1029 nM, $\Delta FRET_{max}= 13$ %).

The following sequences are provided herein:

**Bold:** PDK
Underlined: PS
**Bold and underlined:** CLD
SEQ ID NO: 1
Protein
*Arabidopsis thaliana* CPK21


MGCFSSKHRKTQNDGGEKSIPINPVQTHVVPEHRKPQTPTPKPMTQPIHQQISTPSSNPVS

VRDPDTILGKPFEDIRKF**YSLGKELGRGQFGITYMCKEIGTGNTYACKSILKRKLISKQDKED**

**VKREIQIMQYLSGQPNIVEIKGAYEDRQSIHLVMELCAGGELFDRIIAQGHYSERAAAGIIRSI**

**VNVVQICHFMGVVHRDLKPENFLLSSKEENAMLKATDFGLSVFIEEGKVYRDIVGSAYYVA**

**PEVLRRSYGKEIDIWSAGVILYILLSGVPPFWAENEKGIFDEVIKGEIDFVSEPWPSISESAK**

**DLVRKMLTKDPKRRITAAQVLEHPWI**KGGEAPDKPIDSAVLSRMKQFRAMNKLKKLALKVI**A**

**ESLSEEEIKGLKTMFANIDTDKSGTITYEELKTGLTRLGSRLSETEVKQLMEAADVDGNGTI**

**DYYEFISATMHRYKLDRDEHVYKAFQHFDKDNSGHITRDELESAMKEYGMGDEASIKEVIS**

**EVDTDNDGRINFEEFCAMMRSGSTQ**PQGKLLPFH


SEQ ID NO: 2
Protein
*Arabidopsis thaliana* CPK23


MGCFSSKHRKTQNDGGGERSIPIIPVQTHIVDQVPDHRKPQIPSPSIPISVRDPETILGKPFED

IRKF**YSLGRELGRGGLGITYMCKEIGTGNIYACKSILKRKLISELGREDVKTEIQIMQHLSGQ**

**PNVVEIKGSYEDRHSVHLVMELCAGGELFDRIIAQGHYSERAAAGTIKSIVDVVQICHLNGVI**

**HRDLKPENFLFSSKEENAMLKVTDFGLSAFIEEGKIYKDVVGSPYYVAPEVLRQSYGKEIDI**

**WSAGVILYILLCGVPPFWADNEEGVFVEILKCKIDFVREPWPSISDSAKDLVEKMLTEDPKR**

**RITAAQVLEHPWI**KGGEAPEKPIDSTVLSRMKQFRAMNKLKKLALKVS**AVSLSEEEIKGLKT**

**LFANMDTNRSGTITYEQLQTGLSRLSRLSETEVQQLVEASDVDGNGTIDYYEFISATMHR**

**YKLHHDEHVHKAFQHLDKDKNGHITRDELESAMKEYGMGDEASIKEVISEVDTDNDGKINF**

**EEFRAMMRCGTTQ**PKGKQYPFH


SEQ ID NO: 3
Protein
*Arabidopsis thaliana* CPK1

MGNTCVGPSRNGFLQSVSAAMWRPRDGDDSASMSNGDIASEAVSGELRSRLSDEVQNKP
PEQVTMPKPGTDVETKDREIRTESKPETLEEISLESKPETKQETKSETKPESKPDPPAKPKK
PKHMKRVSSAGLRTESVLQRKTENFKEF**YSLGRKLGQGQFGTTFLCVEKTTGKEFACKSIA**
**KRKLLTDEDVEDVRREIQIMHHLAGHPNVISIKGAYEDVVAVHLVMECCAGGELFDRIIQRG**
**HYTERKAAELTRTIVGVVEACHSLGVMHRDLKPENFLFVSKHEDSLLKTIDFGLSMFFKPD**
**DVFTDVVGSPYYVAPEVLRKRYGPEADVWSAGVIVYILLSGVPPFWAETEQGIFEQVLHGD**
**LDFSSDPWPSISESAKDLVRKMLVRDPKKRLTAHQVLCHPWV**QVDGV<u>APDKPLDSAVLSR</u>
<u>MKQFSAMNKFKKMALRVI**AESLSEEEIAGLKEMFNMIDADKSGQITFEELKAGLKRVGANL**</u>
<u>**KESEILDLMQAADVDNSGTIDYKEFIAATLHLNKIEREDHLFAAFTYFDKDGSGYITPDELQ**</u>
<u>**QACEEFGVEDVRIEELMRDVDQDNDGRIDYNEFVAMMQKGSIT**</u>GGPVKMGLEKSFSIALKL

    SEQ ID NO: 4
    Protein
    *Solanum lycopersicum* CPK1

MGGCFSKKYTQQDANGHRAGRRVNQAYQKPPQPQPERPYQPQPQQERPYQPPPQPAYQ
PPPQPKPQPQPHPVPVTVQSGQPQDQMQGPHMNNILGKPFEEIRKL**YTLGKELGRGQFG**
**VTYYCTENSTGNPYACKSILKRKLVSKNDREDMKREIQIMQHLSGQPNIVEFKGAYEDRQS**
**VHLVMELCAGGELFDRIIARGYYSEKDAAEIIRQIVNVVNICHFMGVMHRDLKPENFLLTSK**
**DENAMLKATDFGLSVFIEEGKVYRDIVGSAYYVAPEVLRRSYGKEADVWSAGVILYILLSG**
**VPPFWAETEKGIFNTILKGEIDFQSDPWPSISNSAKDLIRKMLTQEPRKRITSAQVLEHPWL**
RLGE<u>ASDKPIDSAVLSRMKQFRAMNKLKKLALKVI**AENLSEEEIKGLKAMFHNIDTDNSGTIT**</u>
<u>**YEELKSGLARLGSKLTETEVKQLMEAADVDGNGSIDYIEFITATMHRHRLERDEHLFKAFQ**</u>
<u>**HFDKDHSGFITRDELENAMKEYGMGDEATIKEIIAEVDTDNDGRINYEEFCAMMRSGTTQP**</u>
QQKLF

    SEQ ID NO: 5
    Protein
    *Oryza sativa* CPK1

MGNRTSRHHRAAPEQPPPQPKPKPQPQQQQQQWPRPQQPTPPPAAAPDAAMGRVLGRP
MEDVRAT**YTFGRELGRGQFGVTYLVTHKATGKRFACKSIATRKLAHRDDIEDVRREVQIM**
**HHLTGHRNIVELRGAYEDRHSVNLIMELCEGGELFDRIIARGHYSERAAAALCREIVAVVHS**

**CHSMGVFHRDLKPENFLFLSKSEDSPLKATDFGLSVFFKPGEHFKDLVGSAYYVAPEVLK**
**RNYGAEADIWSAGVILYILLSGVPPFWAESEDGIFDAVLRGHIDFSSEPWPSISNGAKDLVK**
**KMLRQDPKERLTSAEILNHPWI**REDGE<u>APDKPLDITVISRMKQFRAMNKLKKVALKVV**AENL**</u>
<u>**SDEEITGLKEMFRSLDTDNSGTITLEELRSGLPKLGTKISESEIRQLMEAADVDGNGTIDYA**</u>
<u>**EFISATMHMNRLEKEDHILKAFEYFDKDHSGYITVDELEEALKKYDMGDDKTIKEIIAEVDTD**</u>
<u>**HDGRINYQEFVAMMRNNNPE**</u>IAPNRRRMF

SEQ ID NO: 6
Protein
*Selaginella moellendorffii 99178*

MKAAGGIAASTPRSAITASVLGRSTESVREL**YTLGRKLGQGQFGVTYLCVEKSSGKQYACK**

**TIPKRKLISQEDVDDVRREIQIMHHLAGQPNVVQIKGAYEDAGSVHLVMELCAGGELFDRII**

**QRGHYSERKAAELIRVIVGVVQACHSLGVMHRDLKPENFLLLSKHEDSLMKATDFGLSVF**

**FKPGEVFTDVVGSPYYVAPEVLRKKYGPEADVWSAGVILYILLSGVPPFWAETEKGIFEQV**

**LKGEIDFESHPWPVISQDAKDLIRKMLCPVPANRLKAHEVLGHPWA**RADGV<u>APDKPLDSA</u>

<u>VLSRMKQFSAMNKIKKIALRVI</u>**AESLSEEEIAGLKEMFKMMDTDNSGSITFDELKAGLERVG**

**SNLVESEIRDLMAAADVDNSGTIDYKEFITATLHLNKIEREEHLLAAFAYFDKDNSGYITKDE**

**LQQVCAENHMGDEVIEEMMREADQDNDGRIDYSEFVTMMRKGAGG**IGRKTMRNSLSITFR

DLLTV

SEQ ID NO: 7
Protein
*Physcomitrella patens* 1s49_208V6 PpCPK1

MRRGVNLVPGQSFTHSVLQRNTENLKDL**YTLGRKLGQGQFGTTYLCVEKTTGKEYACKSI**

**AKRKLISQEDVDDVRRELHIMHHLSGHPNIVTIKGAYEDQVSVHLVMELCAGGELFDRIIQR**

**GHYSEAQAAELCRVIVGVVETCHSLGVMHRDLKPENFLLSDPSENAALKTTDFGLSVFFK**

**PGEVFTDVVGSPYYVAPEVLRKHYGPEADVWSAGVILYILLSGVPPFWAETEQGIFEQVLA**

**GELDFVSEPWPSISESAKDLIRRMLDPVAKRRLKAHQVLSHPWI**REAGV<u>APDRPMDPAVQ</u>

<u>SRLKQFSAMNKLKKVAIRVI</u>**AEFLSEEEIAGLREMFKMIDTDHSGSITFEELKSGLERVGSNL**

**VESEIRQLMDAADVDQNGTIDYGEFLAATLHLNKIEREENLFAAFSWLDKDHSGYLTVDEL**

**QHACSEYNIGDTSIEELIREVDQDNDGRIDYNEFVTMMRKGNGT**VGRATLRNSLSLSDALM

HTN

SEQ ID NO: 8
Protein
*Chlamydomonas reinhardtii* 33.g782750

MGNCSSQDNTVPAGYKALKVQQVVQQSGDVRDF**YTFDKQLGKGNFGIVHLVFDKKTNEK**

**YACKSISKRKLVTPEDVEDVRREIQIMNHLAGHKNVVNIRGTYEDKNFIHIVMEVGAGGELF**

**DRIAEAGHFSERRAAEVMRTIVSVVHHCHTMNVVHRDLKPENFLLTERGPGGVIKATDFGL**

**SRFFKEGNQLDEIVGSPFYVAPEVLKRSYGKEADIWSCGVILYILLCGWPPFHGDSTQAIFK**

**NILSAPLDLKTEPWSRVSADAKDCVRRMLARDPRKRLTAEQVLNHPWM**RENGA<u>APDEAF</u>

<u>VPEILIRMRQFTKMNMLKREALKVI</u>**ARSLPHMELAGMREMFQEMDEDGSATITVDELREGL**

**RRKGAEIALGEVQRILNDIDLDGNSKIDYEEFLAATMHLNKLSREENMIAAFEYFDKDKSGF**

**ITRDELMNAMKDIDAEVDVDAILAQVDQNGDGRIDYEEFCAMMRATDLD**VLKSAHEALKTK

VVVKSVLARVQAEPMREDSITDMSRKSSRAMATAESRKQRGASATPANAEEGEQ


SEQ ID NO: 9
Protein
*Volvox carteri* 20014333m


MGSCASTENQVPQGYKVLKVQAVVQQQGDVRDY**YTFDKQLGKGNFGIVHLVYDKKTNEK**

**FACKSISKRKLVTSEDVEDVRREIQIMNHLAGHKNIVSIRGTFEDKNFIHIVMELCSGGELFD**

**RIAEAGHFSERRAAEVMRTIVSVVHHCHTMNVVHRDLKPENFLLTERGPGGVIKATDFGLS**

**RFFKEGSSLDEIVGSPFYVAPEVLKRAYGKEADIWSCGVILYILLCGWPPFHGDSTQAIFKN**

**ILSAPLDLKSEPWPRVSPDAKDCVRRMLARDPRKRLTAEQVLNHHWM**RENGA<u>APDEAFV</u>

<u>PEILIRMRQFTKMNLLKREALKVI</u>**ARSLPHMELAGMREMFHDMDEDGSGTITVDELREGLR**

**RKGAEIALSEVQRILNDIDLDGNSKIDYEEFLAATMHLNKLSREENMMAAFEYFDKDKSGFI**

**TRDELVTAMRDIDQEVDVDALLRQVDKNGDGRIDYEEFCLMMRASDLD**VLKCAHEVRILEG


SEQ ID NO: 10
Protein
*Plasmodium falciparum* CDPK1


MGCSQSSNVKDFKTRRSKFTNGNNYGKSGNNKNSEDLAINPGMYVRKKEGKIGES**YFKVR**

**KLGSGAYGEVLLCREKHGHGEKAIKVIKKSQFDKMKYSITNKIECDDKIHEEIYNEISLLKSL**

**DHPNIIKLFDVFEDKKYFYLVTEFYEGGELFEQIINRHKFDECDAANIMKQILSGICYLHKHNI**

**VHRDIKPENILLENKHSLLNIKIVDFGLSSFFSKDNKLRDRLGTAYYIAPEVLRKKYNEKCDV**

**WSCGVILYILLCGYPPFGGQNDQDIIKKVEKGKYYFDFNDWKNISEEAKELIKLMLTYDYNK**

**RITAKEALNSKW**IKKYANNIN<u>KSDQKTLCGALSNMRKFEGSQKLAQAAILFI</u>**GSKLTTLEERK**

**ELTDIFKKLDKNGDGQLDKKELIEGYNILRSFKNELGELKNVEEEVDNILKEVDFDKNGYIE**

**YSEFISVCMDKQILFSEERLRDAFNLFDTDKSGKITKEELANLFGLTSISEQMWNEVLGEAD**

**KNKDNMIDFDEFVNMMHKICDN**KSS


SEQ ID NO: 11
Protein
*Toxoplasma gondii* CDPK1

MGQQESTLGGAAGEPRSRGHAAGTSGGPGDHLHATPGMFVQHSTAIFSDR**YKGQRVLGK**

**GSFGEVILCKDKITGQECAVKVISKRQVKQKTDKES**LLREVQLLKQLDHPNIMKLYEFFED

**KGYFYLVGEVYTGGELF**DEIISRKRFSEVDAARIIRQVLSGITYMHKNKIVHRDLKPENLLLE

**SKSKDANIRIIDFGLSTHFEASKKMKDKIGTAYYIAPEVLHGTYDEKCDVWSTGVILYILLSG**

**CPPFNGANEYDI**LKKVEKGKYTFELPQWKKVSESAKDLIRKMLTYVPSMRISARDALDHE

**WI**QTYTKEQI̲S̲V̲D̲V̲P̲S̲L̲D̲N̲A̲I̲L̲N̲I̲R̲Q̲F̲Q̲G̲T̲Q̲K̲L̲A̲Q̲A̲A̲L̲L̲Y̲M̲**GSKLTSQDETKELTAIFHKMD**

**KNGDGQLDRAELIEGYKELMRMKGQDASMLDASAVEHEVDQVLDAVDFDKNGYIEYSEF**

**VTVAMDRKTLLSRERLERAFRMFDSDNSGKISS**TELATIFGVSDVDSETWKSVLSEVDKNN

**DGEVDFDEFQQMLLKLCGN**

The invention is further illustrated by the following examples, however, without being limited to the example or by any specific embodiment of the examples.

**Examples**

**CPK21 evaluation**

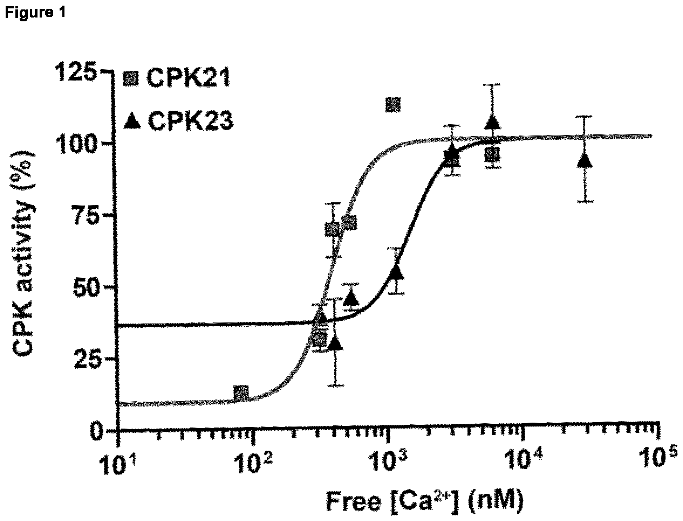

On the basis of *Toxoplasma gondii* CDPK1 structure (Ojo et al., Nat Struct Mol Biol (2010), 17: 602-607; Wernimont et al., Nat Struct Mol Biol (2010), 17: 596-601), a FRET approach was developed where the PS and CLD of CDPKs is sandwiched between a donor (mTurquoise, mT) - and acceptor (Venus circularly permutated at amino acid 173, cpV173) - fluorescence protein pair. The FRET donor was inserted between kinase (PKD) and PS because the kinase domain stabilizes the inactive enzyme conformation via interaction with the PS. Kinase activity measurements with purified CPK21 and a peptide substrate encompassing CPK21 *in vivo* phosphorylation site of slow anion channel-associated 1 (SLAC1) S59 (Geiger (2010), loc. cit.; Brandt et al., eLife (2015), 4: e03599) confirmed a high $Ca^{2+}$-dependent activity with a half maximal activity (K50) of 449 +/- 90 nM $Ca^{2+}$ (Fig. 1). All constructs sandwiching CPK21 PS and CLD displayed enhanced FRET efficiency with increasing [$Ca^{2+}$], and with similar half maximal effective concentration (EC50) for $Ca^{2+}$ (Fig. 11). Variant F4 was used as matrix for all further FRET analyses. The deduced EC50 value for the conformational change was ~ 832 nM $Ca^{2+}$ (Fig. 3). Differences between $Ca^{2+}$-binding-induced conformational change and $Ca^{2+}$-inducible kinase activity indicate that substrate affinity influences $Ca^{2+}$-dependency of activity.

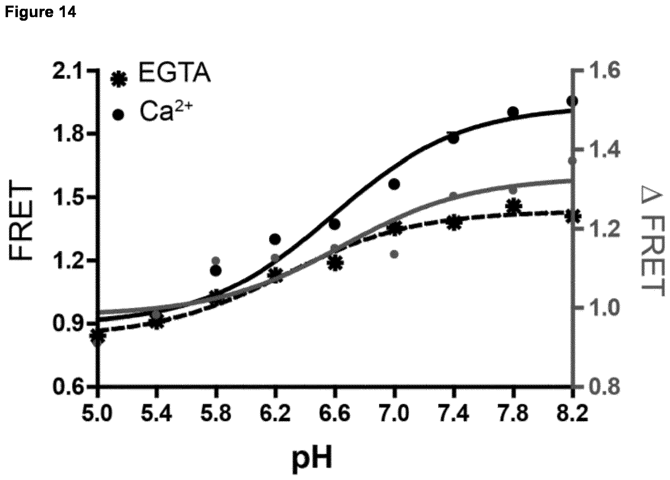

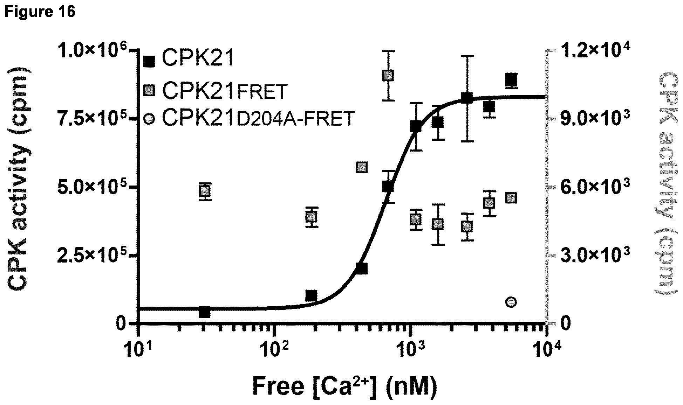

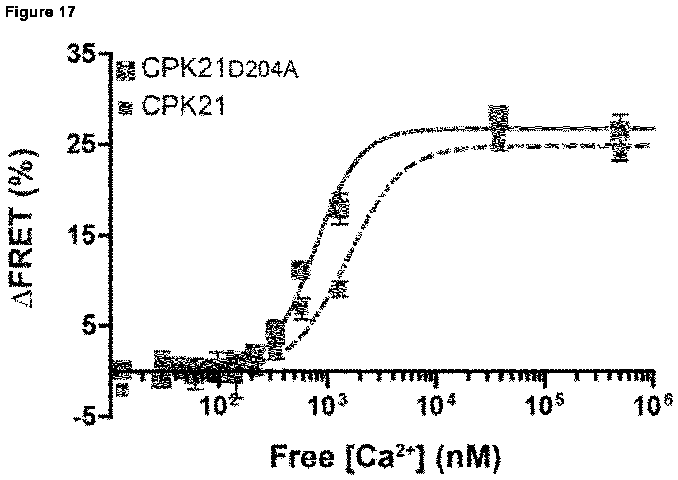

$Ca^{2+}$-induced FRET changes of CPK21D204A were stable over a pH range of ~ 7.2 - 8.2 (Fig. 14). Next, it was tested if intrinsic insertion of mT influences CPK21 activity. CPK21-FRET variant F4 was generated with the active enzyme sequence. CPK21-FRET displayed low constitutive auto- and trans-phosphorylation activity lacking a $Ca^{2+}$-inducible component (Fig. 16). FRET efficiency is similar for active and kinase-deficient CPK21-FRET variants, but activity correlates with an increase of EC50 from 832 nM (CPK21D204A) to 1517 nM $Ca^{2+}$ (Fig. 17).

**CPK23 evaluation**

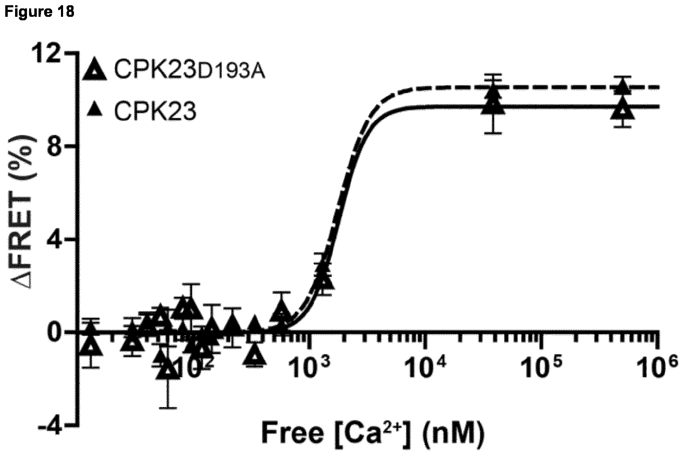

CPK23 activity measurements revealed a composite kinetic with a $Ca^{2+}$-independent core activity of 42 % and a residual $Ca^{2+}$-dependent increase with low affinity (K50 ~1864 nM $Ca^{2+}$) (Fig. 1). Conformational change measurements with kinase deficient CPK23D193A-FRET or native CPK23-FRET showed a weak $Ca^{2+}$-induced alteration in FRET efficiency of 10 or 9 % with a low $Ca^{2+}$-affinity (EC50 ~1391 or ~1533 nM) (Fig. 2, Fig. 18). This demonstrates that differences in CPK21 and CPK23 $Ca^{2+}$ sensitivity of kinase activity are reflected by conformational change. The high percentage of identical amino acids in CPK21 and CPK23 further allows to identify sites that may be responsible for $Ca^{2+}$-dependency. The PS stabilizes CPK conformations through intra-domain interactions. Evaluation of the PS from the A. *thaliana* CPK gene family of 34 members revealed that a conserved isoleucine at consensus PS position 31 is uniquely replaced in CPK23 by serine (S362).

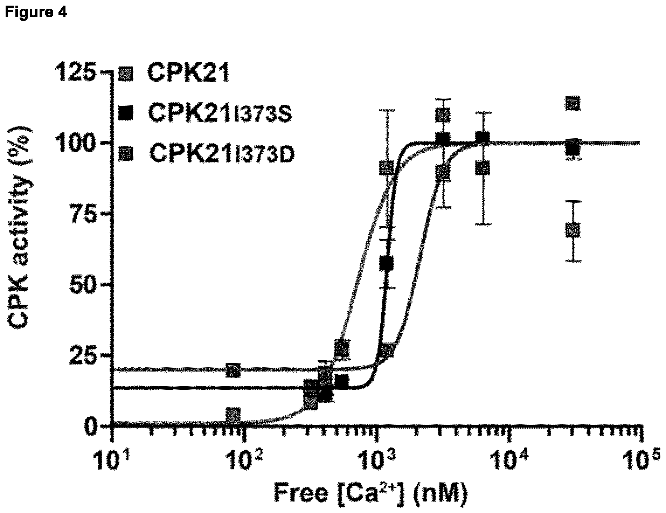

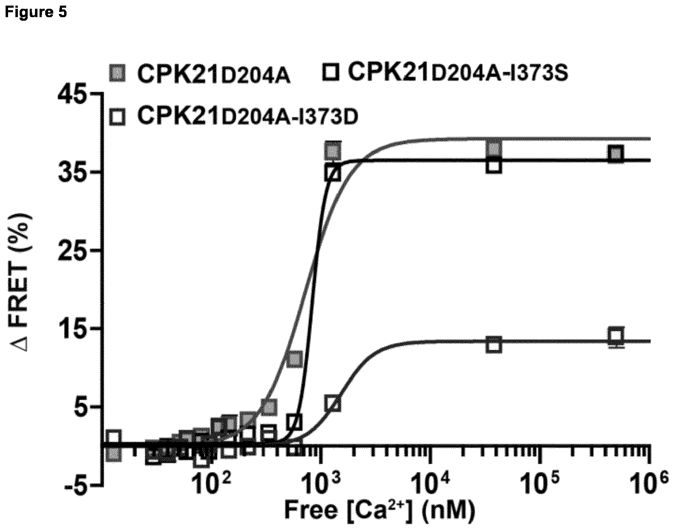

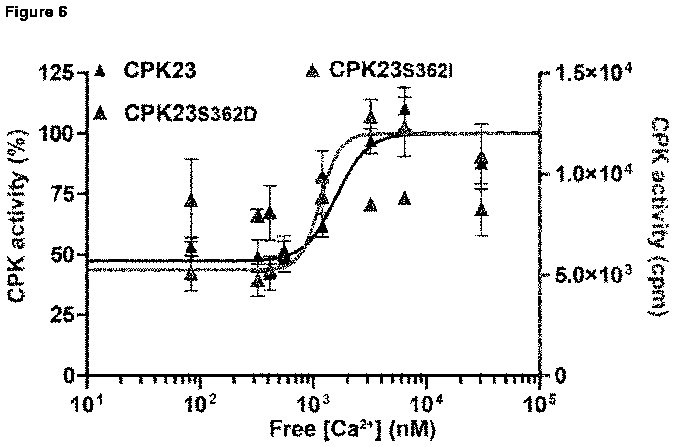

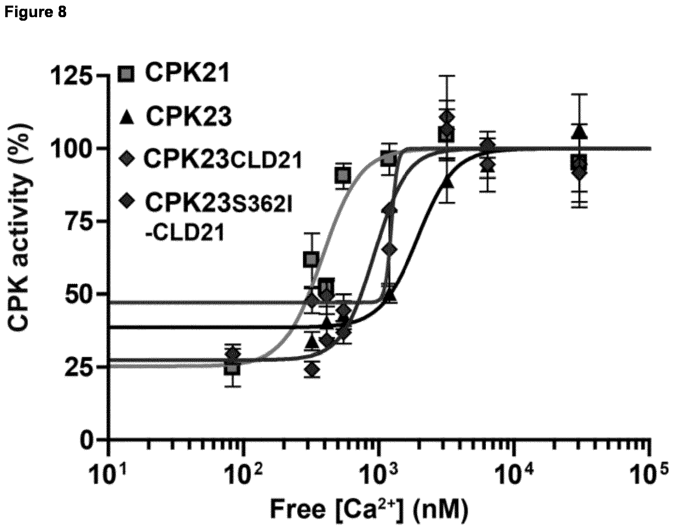

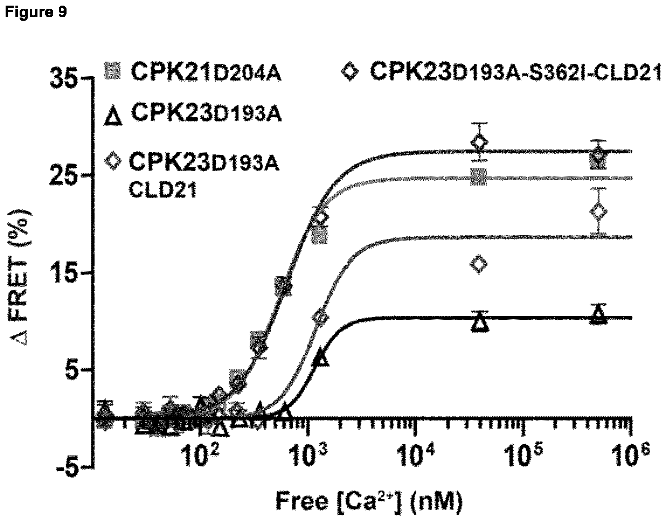

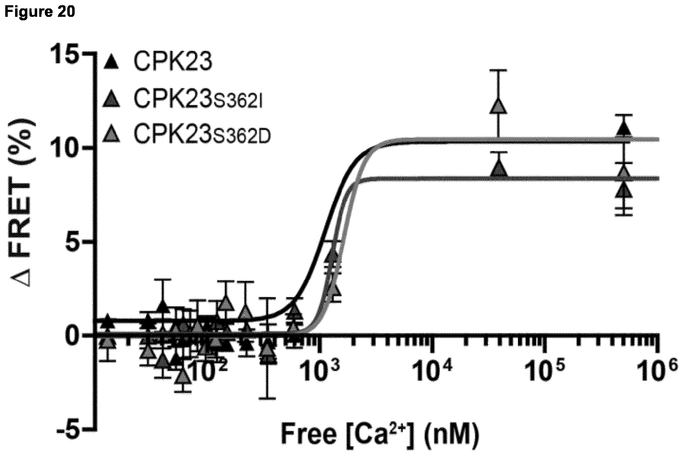

Next, mutually swapped amino acid substitutions at PS position 31 were assessed for their impact on $Ca^{2+}$-dependency of kinase activity and conformational change. In CPK21 substitutions at I373 for S or D, mimicking CPK23, caused an increase of the K50 $Ca^{2+}$ in kinase assays from 449 nM (WT) to 1111 nM (I373S) and 1622 nM (I373D) and an increase of the EC50 $Ca^{2+}$ for the corresponding CPK21D204A-FRET conformational change from 832 nM (D204A) to 925 (D204A-I373S) and 1701 nM (D204A-I373D) (Fig. 4 & 5). These data indicate that the highly $Ca^{2+}$-responsive CPK21 can be turned into the $Ca^{2+}$-insensitive CPK23 upon a single amino acid exchange. In CPK23, the substitution S362I, mimicking CPK21, renders the enzyme in kinase assays more sensitive toward $Ca^{2+}$, and S362D results in constitutive $Ca^{2+}$-independent kinase activity (Fig. 6). Interestingly, when introduced in CPK23-FRET, both amino acid substitutions showed a similar weak conformational change as native CPK23 protein (Fig. 20). But the low FRET efficiency of CPK23 conformational change, compared to CPK21, could mask subtle changes. Therefore, a CLD domain swap was generated. Chimeric CPK23D193A-CLD21-FRET reveals an increase in maximal FRET efficiency whereas the EC50 $Ca^{2+}$ for the conformational change was retained (Fig. 9), indicating that the CLD contributes to the structure of the $Ca^{2+}$-bound form. The combination of both, CLD21 swap and S362I amino acid substitution, in CPK23D193A-CLD21-S362I-FRET, lead to a $Ca^{2+}$-dependency of conformational change indistinguishable from CPK21D204A-FRET (Fig. 9). With respect to kinase activity, CPK23CLD21- and CPK23CLD21-S362I chimeras display increased $Ca^{2+}$-dependency in comparison to native CPK23 (Fig. 8). The substitution at PS position 31 from CPK consensus hydrophobic to polar amino acid (I to S) may impact protein surface properties especially in case of phosphorylated S (mimicked by D). This indicated that ABA-dependent CPK23 autophosphorylation alters the $Ca^{2+}$-inducible component of CPK23 activity. These results identify intramolecular FRET as a tool suitable to determine *in vivo* activation / $Ca^{2+}$-binding / conformational changes of CDPKs.

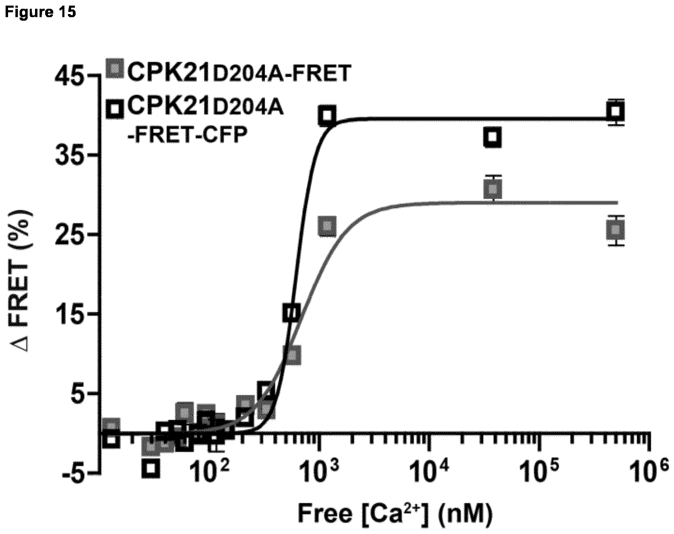

To evaluate the applicability of CDPK-FRET, *in vivo* plant pollen tubes were used which are characterized by their continuous tip-focussed $Ca^{2+}$ gradient essential to maintain polar cell growth. CPK-FRET variants were transiently expressed in pollen tubes that carry the $Ca^{2+}$-sensor R-GECOL (red fluorescent genetically encoded $Ca^{2+}$-indicator for optical imaging) (Zhao et al., Science (2011), 333: 1888-1891) as stable transgene. To increase the brightness of the FRET donor fluorescence, mT was substituted by CFP (cyan fluorescent protein) without altering the kinetics of conformational change (Fig. 15). To focus on cytoplasmic $Ca^{2+}$ concentrations, CPK variants lacking their myristoylation (G2A)- and palmitoylation (C3V)-sites were generated (Simeunovic et al., J Exp Bot (2016), 67: 3855-3872). Pollen tubes display a robust tip-based $[Ca^{2+}]$ oscillation pattern when grown on medium containing 10 mM $Cl^-$. Time-lapse imaging of transfected CPK21G2A-C3V-D204A-FRET-CFP displayed not only a pollen tube tip-focussed FRET signal but recorded the cytosolic $[Ca^{2+}]$ oscillation pattern reported by R-GECO1 simultaneously (data not shown). Thus, reversible CPK21 enzyme activation and inactivation, imaged by CPK-intrinsic FRET, was recorded in real time in its dependency of cytosolic $[Ca^{2+}]$.

### TgCDPK1 (*Toxoplasma gondii*) evaluation

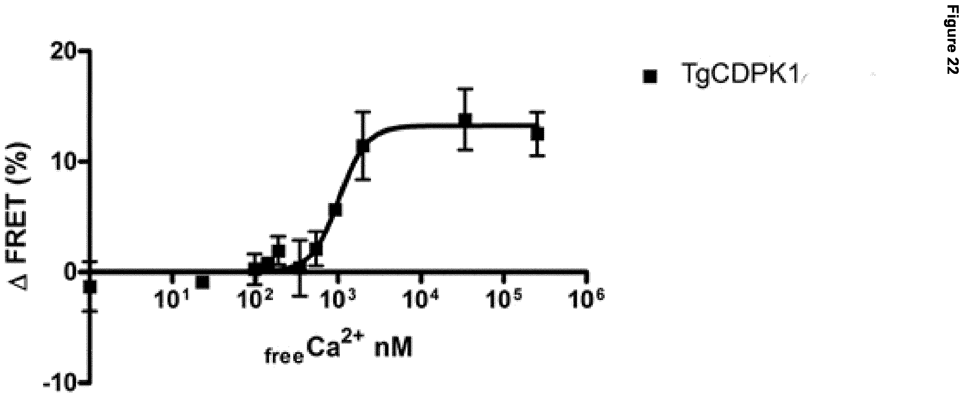

In addition to the existing ratiometric Förster resonance energy transfer (FRET)-based reporter for plant CDPK conformation changes, this approach was extended to the protist CDPK *Toxoplasma gondi* TgCDPK1; cf. also Figure 22.
A construct was created with the donor chromophore (eCFP) inserted C-terminally to amino acid position 308 of SEQ ID NO: 11. The acceptor chromophore was inserted C-terminally of EF4 corresponding to the amino acid position 507 of SEQ ID NO: 11.
The TgCDPK1-FRET fusion protein showed a FRET efficiency change in dependency of $Ca^{2+}$ concentrations. Based on literature data for $Ca^{2+}$-dependent TgCDPK1 kinase activity (Ingram et al., PNAS (2015), E4675-E4984), this shows that TgCDPK1-FRET reflects the conformation change leading to $Ca^{2+}$dependent activation.

### Methods

### Mutagenesis and cloning of CPK21 and CPK23 enzyme variants

Expression vector *pGEX-6P1* (GE Healthcare) -based recombinant synthesis of *CPK21* and *CPK23* constructs carrying a C-terminal polyhistidine-tag and N-terminal GST-tag has been described previously (Geiger (2010), loc. cit.). *CPK21* and *CPK23* in *pGEX-6P1* were used as a template for PCR-based site-directed mutagenesis with specific primers for the variants CPK21I373S, CPK21I3730, CPK23S362I, CPK23S362D (for primer sequence see Table 4).
*CPK23CLD21-S362I* chimeric construct was generated by replacing a part of CPK23 pseudosubstrate segment (from aa 353), the CLD23 and a part of *pGEX-6P1* vector backbone in *pGEX-6P1-CPK23* with the homolog sequence from pGEX-6P1-CPK21 via HindIII and PstI. To create the *pGEX-6P1-CPK23CLD21* construct amino acid substitution I362S was introduced by site-specific mutagenesis primers CPK21I373S-F and CPK21I373S-R with *CPK23CLD21-S362I* in *pGEX-6P1* as PCR template. *CPK23CLD21* and *CPK23CLD21-S362I* chimeric constructs carry the A358 codon from *CPK21* instead of *CPK23* as a result from cloning strategy.
The pXCS-CPK23-HA-Strep *in vivo* expression plasmid used in this study has been described previously (Geiger (2010), loc. cit.). To create a CPK23 kinase-deficient variant, the amino acid substitution D193A was introduced by site-specific mutagenesis primers CPK23D193A-F and CPK23D193A-R.
Mutated coding sequences were verified by sequencing and transferred via restriction sites into the CPK sequence containing vectors.

**Generation of CPK FRET sensors**

*CPK21* and CPK23 variable and kinase domain coding sequences were re-amplified from plasmids pXCS-CPK21D204A-HA-Strep (Geiger (2011), loc. cit.) and *pXCS-CPK23D193A*-HA-Strep using the forward primer CPK21-VK Xbal EcoRI-F or CPK23-VK Xbal EcoRI-F and the reverse primer CPK21/23-VK Xbal-R (for primer sequence see Table 4). Fragments were transferred via the N- and C-terminal introduced Xbal site into pUC-F3-II (Waadt et al., eLife (2014), 3: e01739) resulting in *pUC-F3-II-CPK21D204A*-VK (variable and kinase domain) or *pUC-F3-II-CPK23D193A*-VK. *PUC-F3-II* contain the FRET donor (mTurquoise, mT) (Goedhardt et al., Nat Methods (2010), 7: 137-139) and the FRET acceptor (Venus circularly permutated at amino acid 173, cpV173) (Nagai et al., PNAS (2004), 101: 10554-10559).

*CPK21* pseudosubstrate segment and CLD were isolated by PCR, using for linker variant F1 the primers CPK21-PSCLD Apal-F and CPK21-PSCLD Smal-R, for linker variant F2 CPK21-PSCLD Spel-F and CPK21-PSCLD Kpnl-R, or for linker variant F3 CPK21-PSCLD BamHI-F and CPK21-PSCLD Sall-R and inserted between Apal/Smal (F1), Spel/Kpnl (F2) and BamHI/Sall (F3) in *pUC-F3-II-CPK21D204A*-VK resulting into *pUC-F3-II-CPK21D204A*-FRET (F1-F3), respectively. *CPK21* and *CPK23* coding sequence covering the pseudosubstrate segment and CLD domain until EF-hand 4 (CPK21 aa 522, CPK23 aa 511) was amplified with primers introducing Apal (CPK21-PSCLD Apal-F and CPK23-PSCLD Apal-F) and Smal (CPK21-PSCLD-EF4 Smal-R or and CPK23-PSCLD-EF4 Smal-R) restriction sites and the fragments were inserted between Apal/Smal in *pUC-F3-II-CPK21D204A*-VK to yield *pUC-F3-II-CPK21D204A*-FRET (F4), or in *pUC-F3-II-CPK23D193A*-VK to yield *pUC-F3-II-CPK23D193A*-FRET. CPK21 aa 523 and CPK23 aa 512 was identical to the first aa of Smal restriction site thus in pUC-F3-II-CPK21D204A-FRET (F4), or in pUC-F3-II-CPK23D193A-FRET CPK aa sequence until aa 523 (CPK21) or 512 (CPK23) is present.

*CPK21D204A* full length sequence was amplified using primers CPK21-FL Apal-F and CPK21-PSCLD Smal-R, and the respective fragment was ligated into Apal/Smal of *pUC-F3-II* resulting in pUC-F3-II-CPK21D204A-FRET (F5).

*Escherichia coli* expression vectors were obtained by sub-cloning CPK21D204A-FRET (F1-F4) and CPK23D193A-FRET via EcoRI/Sacl or CPK21D204A-FRET (F5) via Ndel/Sacl into pET-30a (+) (Novagen). The resulting *pET-30a-CPK21D204A*-FRET (F1-F4) and *pET-30a*-CPK23D193A-FRET constructs carry a N-terminal polyhistidine-tag derived from *pET-30a* vector backbone and a C-terminal Strep-tag derived from pUC-F3-II-*CPK21D204A*-FRET (F1-F4) or *pUC-F3-II-CPK23D193A*-FRET constructs. *pET-30a-CPK21D204A*-FRET (F5) carries a Strep-tag derived from *pUC-F3-II-CPK21D204A*-FRET (F5).

For cloning of *CPK21*- and CPK23-FRET variants, mutation-containing CPK sequences or the CPK23CLD21 chimeric sequence were exchanged via restriction sites from pGEX-6P-1-CPK constructs into pET30a-CPK-FRET.

For *in vivo* transient expression CPK21D204A-FRET and *CPK23D193A*-FRET were subcloned via EcoRI/Ecl136ll and inserted between EcoRI/Sfol into *pXCS-HA-Strep* (Witte et al., Plant Mol Biol (2004), 55: 135-147) yielding *pXCS-CPK21D204A*- or *pXCS-CPK23D193A*-FRET. The p35S of pXCS-CPK-FRET was substituted with the *ubiquitin4-2* promoter from parsley from V69-Ubi:Cas9-MCS-U6 (Kirchner et al., PLoS ONE (2017), 12: e0185429) via Ascl/Xhol yielding *pXC-Ubi-CPK21D204A-FRET* or *pXC-Ubi-CPK23D193A-FRET.* For cytosolic localization, *pXC-Ubi-CPK-FRET* construct was used as a template for PCR-based site-directed mutagenesis introducing G2A in a first mutagenesis reaction and G2A-C3V mutation in a second reaction (for primer sequence see Table 4) (Wener et al., Gene (1994), 151: 119-123). Mutated coding sequences were validated by sequencing and transferred via restriction sites within CPK-VK sequence into the *pXC-Ubi-CPK-FRET* construct.

The primers CFP Ndel-F and CFP Apal-R (Table 4) were used to amplify CFP (cyan fluorescent protein) (Heim et al., Curr Biol (1996), 6: 178-182) with attached Ndel/Apal sites and CFP was cloned into Ndel and Apal linearized *pXC-Ubi-CPK21G2A-C3V*-FRET or *pXC*-Ubi-CPK23G2A-C3V-FRET resulting in pXC-Ubi-CPK21G2A-C3V-FRET-CFP or *pXC-Ubi-CPK23G2A-C3V-FRET-CFP.*

**Expression in *Escherichia coli* and protein purification**

CPK constructs were in general synthesized as recombinant double-tagged fusion proteins in *E. coli.* For *in vitro* kinase assays expression vector *pGEX-6P-1* was used and proteins were purified using the N-terminal His-Tag and a C-terminal GST-Tag. For *in vitro* FRET measurements, expression vector *pET30a-CPK-FRET* was used and proteins were purified using the N-terminal His-Tag and a C-terminal Strep-Tag.

To synthesize and purify proteins for kinase assays the *pGEX-6P-1-CPK* expression, vectors were introduced in *E. coli* BL21 (DE3) (Stratagene). Bacteria were grown at 37 °C in LB medium containing 100 $\mu$g/ml ampicillin and protein expression was induced at an $OD_{600}$ of 0.4 - 0.6 with 0.3 mM isopropylthiol-$\beta$-galactoside (IPTG). Cells were incubated for additional 4 h at 28 °C and harvested by centrifugation. Cells were lysed in 4 ml histidine-lysis buffer (50 mM HEPES-KOH pH 7.4, 300 mM NaCl, 0.2 % (v/v) Triton X-100, 1 mM DTT, 10 $\mu$l protease inhibitor cocktail for histidine tagged proteins (Sigma) / 0.2 g weight of *E.coli* cells and 30 mM imidazole) using 1°mg/ml lysozyme and sonification and a centrifugation was performed to remove the cell debris. Supernatant was gently end-over-end rotated with 300-600

μl Ni sepharose 6 fast flow (GE Healthcare) at 4°C for 1 h. Sample/Ni sepharose mix was loaded on empty columns and washed 1 × 10 ml histidine-washing buffer (50 mM HEPES-KOH pH 7.4, 300 mM NaCl) with 30 mM imidazole and 1 × 10 ml histidine-washing buffer with 40 mM imidazole. Proteins were eluted 3 × in 500 μl histidine-elution buffer (50 mM HEPES-KOH pH 7.4, 300 mM NaCl, 500 mM imidazole). Eluate was incubated at 4 °C for 1 h with Glutathione sepharose. Eluate/Glutathione sepharose mix was loaded on empty columns washed 3 × 3 ml GST-wash buffer (50 mM Tris-HCl pH 8.0, 250 mM NaCl, 1 mM DTT) and eluted 3 × with 300 μl GST-elution buffer (100 mM Tris-HCl pH 8.4 and 20 mM glutathione). Proteins were dialyzed using micro dialysis capsule QuixSep (Roth) and dialysis membrane with 6000 - 8000 Da cut off (Roth). Dialysis-buffer was composed of 30 mM MOPS pH 7.4 and 150 mM KCl.

To synthesize and purify proteins for FRET measurements, *pET30a-CPK-FRET* expression vectors were transformed into *E. coli* BL21 (DE3) pLySs strain (Stratagene). Bacteria were grown at 37 °C in TB medium containing 50 μg/ml kanamycin and 34 μg/ml chloramphenicol and protein expression was induced at an $OD_{600}$ of 0.4 - 0.6 with 0.4 mM IPTG. Cells were incubated for additional 4 h at 22 °C and harvested by centrifugation. Cells were lysed in 6 ml histidine-lysis buffer (see above) using 1 mg/ml lysozym and sonification followed by centrifugation to remove cell debris. Supernatant was incubated with 300 - 600 μl Ni sepharose 6 fast flow (GE Healthcare) at 4°C for 1 h. Sample/Ni sepharose mix was loaded on empty columns and washed 1 × 10 ml histidine-washing buffer with 30 mM imidazole and 1 × 10 ml histidine-washing buffer with 40 mM imidazole. Proteins were eluted 3 × in 500 μl histidine-elution buffer. Eluate was incubated at 4°C for 45 min with Strep-tactin macroprep (IBA). Strep-tagged recombinant proteins were purified as described by Schmidt and Skerra (Schmidt et al., Nat Protoc (2007), 2: 1528-1535) with the modification that EDTA was omitted from elution and wash buffer. Proteins were dialyzed using micro dialysis capsule QuixSep (Roth) and dialysis membrane with 6000 - 8000 Da cut off (Roth). Dialysis buffer was composed 30 mM Tris-HCl pH 7.4, 150 mM NaCl and 10 mM $MgCl_2$.

The CPK21D204A-FRET variant containing the entire CPK flanked by the fluorescence proteins (F5) was affinity purified using the C-terminal Strep-Tag as described (Schmidt, loc. cit.) with the modification that EDTA was omitted from elution and wash buffer.

Protein purity of *E. coli* expressed proteins was confirmed by 10 % SDS-PAGE and Coomassie staining. For *in vitro* analyses, protein concentrations were quantified based on the method of Bradford (Protein assay, Bio-Rad).

## Protein sequence comparison

The analysis of the pseudosubstrate segment of the entire *A. thaliana* Col-0 CPK gene family was conducted on basis of protein sequences from uniport (The UniProt Consortium, Nucleic Acid Res (2017), 45: D158-D169) with the program Web Logo (Crooks et al., Genome Res (2004), 14: 1188-1190; Schneider et al., Nucleic Acid Res (1990), 18: 6097-6100).

## Preparation of calcium and magnesium buffers

For CPK protein kinase assays, calculated reciprocal dilutions of zero-$Ca^{2+}$-buffer (10 mM EGTA 150 mM KCl, 30 mM MOPS pH 7.4) with high-$Ca^{2+}$-buffer (10 mM $CaCl_2$, 10 mM EGTA 150 mM KCl, 30 mM MOPS pH 7.4) were mixed. For the analysis of CPK-FRET conformational changes high-$Ca^{2+}$-buffer (20 mM $CaCl_2$; 20 mM EGTA, 150 mM NaCl, 10 mM $MgCl_2$, 30 mM Tris-HCl pH 7.4) and zero-$Ca^{2+}$-buffer (20 mM EGTA, 150 mM NaCl, 10 mM $MgCl_2$, 30 mM Tris-HCl pH 7.4) were mixed accordingly, preceding a 1:1 dilution with CPKs. Correspondingly, buffer solutions for CPK-FRET analysis in a $Mg^{2+}$ concentration gradient were prepared by a mixture of high-$Mg^{2+}$-buffer (120 mM $MgCl_2$, 20 mM EDTA, 150 mM NaCl, 30 mM Tris-HCl pH 7.4) and zero-$Mg^{2+}$-buffer (20 mM EDTA, 150 mM NaCl, , 30 mM Tris-HCl pH 7.4), followed by a 1:1 dilution with FRET protein in $Mg^{2+}$-dialysis buffer (30 mM Tris-HCl pH 7.4, 150 mM NaCl) before data acquisition. The indicated free $Ca^{2+}$ or $Mg^{2+}$ concentrations were calculated with the WEBMXC extended website http://www.stanford.edu/~cpatton/maxc.html based on Patton et al., Cell Calcium (2004), 35: 427-431.

## *In vitro* kinase assays

*In vitro* kinase activity with recombinant purified proteins were conducted as described using a 20 aa peptide (41-RGPNRGKQRPFRGFSRQVSL-60; JPT Peptide Technologies) derived from the CPK21 and CPK23 *in vivo* phosphorylation substrate protein SLAC1 (slow anion channel-associated 1). For kinase reaction (30 μl) the enzyme (~90 nM) was incubated in 25 mM MOPS pH 7.4, 125 mM KCl, 10 mM $MgCl_2$, 10 μM ATP, 3 μCi [γ-$^{32}$P]-ATP, 10 μM SLAC1 peptide, 6.67 mM EGTA and different concentration of $CaCl_2$ for 20 min at 22 °C. The reaction was stopped by adding 3 μl 10 % phosphoric acid. Phosphorylation of the SLAC1 peptide was assessed after binding of phosphor-peptides to P81 filter paper and scintillation counting as described (Franz et al., Mol Plant (2011), 79: 803-820). 2 - 4 technical replicates were evaluated per sample. $Ca^{2+}$-dependent kinase activity is indicated as percentage of maximal activity (best fit-value obtained for maximal activity). Kinase activities can be described by a four parameter logistic equation with a global model with shared Hillslope (GraphPad Prism Software). For comparison of enzyme variants data from different measurements were combined in one figure.

For analysis of autophosphorylation activities of protein kinases, the reaction was the same as above with the modifications that SLAC1 substrate peptide was omitted from kinase reaction and -255 nM enzyme was used. The reaction was stopped by adding 5 × SDS-PAGE loading buffer and boiling for 5 min and samples were separated by 10% SDS-PAGE. Phosphorylation was determined by autoradiography and phospho-imaging (Typhoon FLA 9500, GE Healthcare).

### *In vitro* analyses of CPK-FRET

CPK-FRET protein (~415 nM) in dialysis buffer diluted 1:1 with $Ca^{2+}$-buffers of defined concentrations (see above) was evaluated using the TECAN Infinite M200 PRO plate reader (TECAN). Excitation at 435 nm (bandwidth 5 nm) and emission within the range of 470 - 600 nm was monitored in 2 nm steps with 10 flashes of 20 $\mu$s and 400 Hz. To obtain optimal emission spectra the gain settings were calculated by the TECAN software from a CPK-FRET high-$Ca^{2+}$-buffer sample. cpVenus173/mTurquoise FRET ratios were calculated on the basis of maximal values from emission bands of mTurquoise (470 - 490 nm) and cpVenus173 (518 - 538 nm). FRET ratios are plotted against increasing $Ca^{2+}$ concentrations using GraphPad Prism 4 software and are described by a four parameter logistic equation with shared Hillslope value for all data sets of the same enzyme. The best fit-value obtained for bottom FRET ratio is used to calculate ΔFRET as the percentage change of emission ratios. ΔFRET is defined as:

$$\Delta FRET \left[ _{free} Ca^{2+} \right]_{x\ \mu M} = \frac{FRET\ ratio\ \left[ Ca^{2+} \right]_{x\ \mu M}}{FRET\ ratio\ Bottom} \times 100 - 100$$

The percentage change of emission ratio ΔFRET is fitted by a four parameter logistic equation using graph GraphPad Prism 4 software. For comparison of enzyme variants, data from different measurements were combined in one figure.

The pH-dependency of a CPK-FRET sensor was assessed with CPK21D204A-FRET in dialysis buffers (30 mM Tris-HCl, 150 mM NaCl and 10 mM $MgCl_2$) of different pH values (pH 5.0, 6.9 and 8.0). Dialysed proteins were diluted 1:1 with either high-$Ca^{2+}$-buffer or zero-$Ca^{2+}$-buffer (see above) within 30 mM Tris-HCl adjusted accordingly to a pH range between 5.0 - 8.4. Curves were fitted by a four parameter logistic equation and ratio change was calculated by dividing the $Ca^{2+}$ through the EGTA value.

### Protein expression in protoplast and purification for MS measurement

Preparation and transfection of Arabidopsis leaf mesophyll protoplasts to transiently express CPK23-HA-Strep and CPK23D193A-HA-Strep was conducted as described (Wu et al., Plant Methods (2009), 5: 16). Approximately 9.6 × 10^5 protoplasts of cpk23 (SALK_007958) (Ma et al., Plant Mol Biol (2007), 65: 511-518) line were transfected with 96 $\mu$g of pXCS-CPK23-HA-Strep or pXCS-CPK23D193A-HA-Strep and incubated in the dark for 14 h. One transfection reaction was divided by two for ABA treatment and as control. Protoplasts were treated with 30 $\mu$M final ABA for 10 min at RT. Cells were collected by centrifugation (twice 10000 × g, 2 s), frozen in liquid nitrogen, and pellets were stored at - 80 °C. Protoplasts were resuspended into 600 $\mu$l of extraction buffer (100 mM Tris-HCl pH 8.0, 100 mM NaCl, 5 mM EDTA, 5 mM EGTA, 20 mM DTT, 10 mM NaF, 10 mM $NaVO_4$, 10 mM $\beta$-glycerole-phosphate, 0.5 mM AEBSF, 2 $\mu$g/ml aprotinin, 2 $\mu$g/ml leupeptin, 100 $\mu$g/ml avidin, 0.2% NP-40, 1 × phosphatase inhibitor mixture (Sigma) and 1 × protease inhibitor mixture (Sigma) and centrifuged at 20000 × g for 10 min at 4 °C. Supernatant was incubated with 24 $\mu$l Strep-Tactin MacroPrep (IBA) beads on a rotation wheel for 45 min at 4 °C. After centrifugation (500× g, 1 min) beads were solved in 100 $\mu$l 6 M urea, 2 M thiourea, pH 8.0 and incubated for 10 min at 4 °C on a rotation wheel. After centrifugation (500 × g, 1 min) the protein containing supernatant was transferred to a new tube and reduction of disulfide bonds, alkylation of cysteines and tryptic digestion was conducted as described (Dubiella et al., PNAS (2013), 110: 8744-8749). Peptide containing reactions were vacuum-dried at 30 °C and stored at-20 °C.

### Targeted analysis phosphorylation by directed MS

Samples were subsequently desalted through C18 tips. Digested protein mixtures were spiked with 500 fmol of 13C$_6$-R/K mass-labelled standard peptide before mass spectrometry analysis. Tryptic peptide mixtures including the stable-isotope labelled standard peptides were analysed on a nano-HPLC (Easy nLC, Thermo Scientific) coupled to an Orbitrap mass spectrometer (LTQ-Orbitrap, Thermo Scientific) as mass analyser. Peptides were eluted from a 75 $\mu$m analytical column (Easy Columns, Thermo Scientific) on a linear gradient running from 10 % to 30 % acetonitrile in 120 min and were ionized by electrospray.

The target peptide V(pS)AVSLSEEEIK (m/z of doubly-charged ion for phosphopeptide 685.8262; non-phosphopeptide 645.8430) was analysed in its phosphorylated and non-phosphorylated state using the stable-isotope labelled synthetic standard peptide as an internal reference and for normalisation between samples. Standards carried a $^{13}C_6$-labeled amino acid (arginine or lysine) at their C-terminal ends.

Information-dependent acquisition of fragmentation spectra for multiple-charged peptides was used with preferred precursor selection of the target peptides through implementation of an inclusion lists (Schmidt et al., Mol Syst Biol (2011), 7: 510). Full scans were obtained at a resolution of FWHM (full width at half maximum) of 60000, CID fragment spectra were acquired in the LTQ. Additional fragmentation though multistage activation (Schroeder et al., Anall Chem (2004), 76: 3590-3598) was used if peptides displayed a loss of phosphoric acid (neutral loss, 98 Da) upon MS/MS fragmentation.

Protein identification and intensity quantitation was performed as described (Menz et al., Plant J (2016), 88: 717-734). To allow robust identification and quantitation of the internal standard peptide, multiplicity was set to 2 and Lys6 and Arg6 were selected as stable isotope labels and in general, data analysis was focused on the target peptide sequences only.

### Quantification of target peptide abundance changes

For quantitative analysis, the ion intensities of $^{13}C_6$-labeled standard peptides were used for normalisation between samples and replicates. Normalised ion intensities of phosphorylated and non-phosphorylated target peptides were averaged between replicates of the same treatments.

### Transient pollen transformation

*Nicotiana tabacum* (cultivars Petit Havana SR1) plants were grown on soil with a day/night regime of 10 h/14 h, and a temperature of 22 to 24/20 to 22°C provided by a 30 klx white light (SON-T Agro 400W; Philips). Pollen of tobacco lines expressing the R-GECOL calcium sensor (Zhao, loc. cit.) as a stable transgene under the control of a pollen- specific promoter (pLeLAT52::R-GECO1 line) was used from frozen stocks to perform transient transformation using a homemade particle bombardment device has been described in detail (Gutermuth et al., Plant Cell (2013), 25: 4525-4543). Biolistic transformation was performed with pXC-Ubi-CPK21D204A-G2A-C3V-CFP and pXC-Ubi-CPK23D193A-G2A-C3V-CFP on agar plates containing pollen tube growth medium (1 mM MES-Tris pH 5.8, 0.2 mM $CaCl_2$, 9.6 mM HCl, and 1.6 mM $H_3BO_3$). Osmolarity of pollen media was adjusted to 400 mosmol kg$^{-1}$ (Vapor Pressure Osmometer 5520) with D(+)-sucrose.

### Live-cell fluorescence imaging

The setup for wide-field live-cell imaging and the appropriate software to control sample acquisition has been described in detail (Guthermuth, loc. cit.). Images were recorded with a time interval of 5 s. For simultaneous CFP/YFP/RFP-imaging a triple-band dichroic mirror (Chroma # 69008; ET - ECFP/EYFP/mCherry) was used to reflect excitation light on the samples. Excitation of CFP and R-GECOL was performed with a VisiChrome High-Speed Polychromator System (Visitron Systems) at 420 nm and 550 nm, respectively. Optical filters (Chroma Technology Corporation) for CFP (ET 470/24 nm), YFP (ET 535/30 nm) and R-GECOL (624/40) were used for fluorescence detection with a back-illuminated $512 \times 512$ pixel Evolve EMCCD camera (Photometrics). A high-speed 6-position filter wheel (Ludl Electronic Products Ltd.) ensured the quasi simultaneous imaging of all three channels with a lag-time of -0.1 sec. For image processing the following steps were conducted for R-GECO (R-GECO$_{excitation}$/R-GECO$_{emission}$), FRET (CFP$_{excitation}$/YFP$_{emission}$) and CFP (CFP$_{excitation}$/CFP$_{emission}$) channels using Fiji (National Institute of Health) background subtraction (same value for FRET and CFP channel), gaussian blur, 32-bit conversion, kymographs were generated, and threshold adjusted. A self-made script for the Octave 4.0.3 free software http://www.gnu.org/software/octave/) was used to quantify fluorescence intensities of each channel at ~5 - 15 $\mu$m behind the tip of the growing pollen tubes over time. FRET-analysis was performed by dividing the FRET signal by the CFP signal. Correlation analyses for R-GECO and FRET channels signals were done using graph GraphPad Prism 4 software.

### Statistics

Analyses of kinase activities and conformational changes were performed by a four parameter logistic equation with a global model with shared hillslope for all data sets of the same enzyme. A 95% confidence interval was used. Statistical analysis of MS-data was performed by one-way ANOVA (P = 0.005, F = 9.604, degrees of freedom = 3 (between columns) and = 8 (within columns)) followed by Tukey's multiple comparison test and P < 0.05 was considered significant. For Pearson correlation a two-tailed P value, a 95% confidence interval and a significance level of 0.05 was used. All statistical analyses were performed using GraphPad Prism 4.

**Table 4:** Sequences of oligonucleotide primers

| Construct | Sequence | SEQ ID NO. |
|---|---|---|
| **site-directed mutagenesis primers** | | |
| CPK21D204A-F | GGTGTGGTTCATCGAGCTCTCAAGCCTGAG | 12 |
| CPK21D204A-R | CTCAGGCTTGAGAGCTCGATGAACCACACC | 13 |
| CPK23D193A-F | GTGTGATTCATCGAGCTCTCAAGCCTGAG | 14 |
| CPK23D139A-R | CTCAGGCTTGAGAGCTCGATGAATCACAC | 15 |
| CPK21I373S-F* | GCTCTAAAGGTTAGCGCGGAGAGTCTATC | 16 |
| CPK21I373S-R* | GATAGACTCTCCGCGCTAACCTTTAGAGC | 17 |
| CPK21I373D-F | GCTAGCTCTAAAGGTTGACGCGGAGAGTCTATC | 18 |
| CPK21I373D-R | GATAGACTCTCCGCGTCAACCTTTAGAGCTAGC | 19 |
| CPK23S362I-F | GCCCTAAAGGTTATCGCGGTGAGTCTATC | 20 |
| CPK23S362I-R | GATAGACTCACCGCGATAACCTTTAGGGC | 21 |
| CPK23S362D-F | GCCCTAAAGGTTGACGCGGTGAGTCTATC | 22 |
| CPK23S362D-R | GATAGACTCACCGCGTCAACCTTTAGGGC | 23 |
| CPK21G2A-F | GATATCGAATTCATGGCTTGCTTCAGCAGTAAACAC | 24 |
| CPK21G2A-R | GTGTTTACTGCTGAAGCAAGCCATGAATTCGATATC | 25 |
| CPK21 G2AC3V-F | GATATCGAATTCATGGCTGTCTTCAGCAGTAAACAC | 26 |
| CPK21 G2AC3V-R | GTGTTTACTGCTGAAGACAGCCATGAATTCGATATC | 27 |
| CPK23G2A-F | GATATCGAATTCATGGCTTGTTTCAGCAGTAAACAC | 28 |
| CPK23G2A-R | GTGTTTACTGCTGAAACAAGCCATGAATTCGATATC | 29 |
| CPK23G2AC3V-F | GATATCGAATTCATGGCTGTTTTCAGCAGTAAACAC | 30 |
| CPK23G2AC3V-R | GTGTTTACTGCTGAAAACAGCCATGAATTCGATATC | 31 |
| **Cloning of CPK-FRET constructs** | | |
| CPK21-FL Apal-F | ATGGGCCCATGGGTTGCTTCAGCAG | 32 |
| CPK21-VK Xbal EcoRI-F | ATTCTAGA GAATTCATGGGTTGCTTC | 33 |
| CPK23-VK Xbal EcoRI-F | ATTCTAGA GAATTCATGGGTTGTTTC | 34 |
| CPK21/23-VK Xbal-R | ATTCTAGATTCTCCCCCTTTGATCCAAG | 35 |
| CPK21-PSCLD Apal-F | ATGGGCCCGCACCAGACAAGCCTATTG | 36 |
| CPK21-PSCLD Spel-F | ATACTAGTGCACCAGACAAGCCTATTG | 37 |
| CPK21-PSCLD BamHI-F | GGATCCGCACCAGACAAGCCTATTG | 38 |
| CPK21-PSCLD Smal-R | CCCGGGATGGAATGGAAGCAGTTTC | 39 |
| CPK21-PSCLD Kpnl-R | ATGGTACCATGGAATGGAAGCAGTTTC | 40 |
| CPK21-PSCLD Sall-R | ATGTCGACATGGAATGGAAGCAGTTTC | 41 |
| CPK21-PSCLD-EF4 Smal-R | ATCCCGGGCTGCGTGCTGCCACTTC | 42 |
| CPK23-PSCLD-EF4 Smal-R | ATCCCGGGTTGTGTGGTGCCACATC | 43 |
| CFP Ndel-F | ATCATATGGTGAGCAAGGGCGAGG | 44 |

(continued)

| Cloning of CPK-FRET constructs | | |
|---|---|---|
| CFP Apal-R | ATGGGCCCCTTGTACAGCTCGTCCATG | 45 |
| * Used for mutagenesis of I362 in CPK23CLD21-S362I yielding in CPK23CLD21<br>F: forward, R: reverse | | |

## Claims

1. Calcium dependent protein kinase (CDPK) polypeptide comprising a variable domain (VD), a protein kinase domain (PKD), a pseudosubstrate segment (PS), and a calmodulin-like domain (CLD), said CLD comprising 4 EF-hand motifs EF1, EF2, EF3 and EF4,
   wherein

   (A) a donor chromophore is inserted between PKD and PS and an acceptor chromophore is inserted C-terminally of EF4, or
   (B) an acceptor chromophore is inserted between PKD and PS and a donor chromophore is inserted C-terminally of EF4,

   wherein said donor chromophore and said acceptor chromophore represent a Förster Resonance Energy Transfer (FRET) pair.

2. Polypeptide of claim 1, wherein said donor chromophore and/or said acceptor chromophore is a fluorescent protein (FP).

3. Polypeptide of claim 1 or 2, wherein

   (a) said donor chromophore is a cyan FP (CFP), and said acceptor chromophore is a yellow FP (YFP),
   (b) said donor chromophore is a green FP (GFP) or yellow FP (YFP), and said acceptor chromophore is a red FP (RFP),
   (c) said donor chromophore is a cyan FP (CFP), and said acceptor chromophore is a green FP (GFP),
   (d) said donor chromophore is a red FP (RFP), and said acceptor chromophore is a near-infrared FP, or
   (e) said donor chromophore is a non-naturally occurring amino acid and said acceptor chromophore is an FP.

4. Polypeptide of any one of claims 1 to 3, wherein

   (i) said donor chromophore is selected from the group consisting of mTurquoise (mT), eCFP, Aquamarine, mTurquoise2 (mT2), mCerulean3, mTFP1 and LUMP, and said acceptor chromophore is selected from the group consisting of cpVenus173, eYFP, mVenus, Venus, mCitrine, sEYFP and YPet, or
   (ii) said donor chromophore is selected from the group consisting of cpVenus173, eGFP, NowGFP, Clover, mClover3 and mNeonGreen, and

   said acceptor chromophore is selected from the group consisting of mRuby2, mRuby3, mRFP, nKOk, and mCherry.

5. Polypeptide of any one of claims 1 to 4, wherein said donor chromophore is mT or eCFP, and said acceptor chromophore is cpVenus173.

6. Polypeptide of any one of claims 1 to 5, wherein said donor chromophore is inserted directly at the C-Terminus of the PKD, directly at the N-Terminus of the PS, or between the C-Terminus of the PKD and the N-Terminus of the PS.

7. Polypeptide of any one of claims 1 to 6, wherein said acceptor chromophore is inserted directly at the C-Terminus of EF4, at the C-Terminus of the CDPK polypeptide, or between the C-Terminus of EF4 and the C-Terminus of the CDPK polypeptide.

8. Polypeptide of any one of claims 1 to 7, wherein the amino acid sequence comprising the PKD, the PS and the CLD of the CDPK is at least 37% identical to amino acid sequence 80 to 522 of SEQ ID NO: 1, 69-511 of SEQ ID NO:

2, 150-592 of SEQ ID NO: 3, 105-547 of SEQ ID NO: 4, 66-509 of SEQ ID NO: 5, 32-474 of SEQ ID NO: 6, 29-471 of SEQ ID NO: 7, 34-475 of SEQ ID NO: 8, 34-475 of SEQ ID NO: 9, 56-521 of SEQ ID NO: 10, or 51-507 of SEQ ID NO: 11.

9. Polypeptide of any one of claims 1 to 8, wherein the amino acid sequence comprising the PKD, the PS and the CLD of the CDPK is at least 54% similar to amino acid sequence 80-522 of SEQ ID NO: 1, 69-511 of SEQ ID NO: 2, 150-592 of SEQ ID NO: 3, 105-547 of SEQ ID NO: 4, 66-509 of SEQ ID NO: 5, 32-474 of SEQ ID NO: 6, 29-471 of SEQ ID NO: 7, 34-475 of SEQ ID NO: 8, 34-475 of SEQ ID NO: 9, 56-521 of SEQ ID NO: 10, or 51-507 of SEQ ID NO: 11.

10. Polypeptide of any one of claims 1 to 9, wherein

(1) the amino acid sequence comprising the PKD is at least

44% identical to the amino acid sequence 80 to 338 of SEQ ID NO: 1, 69-327 of SEQ ID NO: 2, 150-408 of SEQ ID NO: 3, 105-363 of SEQ ID NO: 4, 66-324 of SEQ ID NO: 5, 32-290 of SEQ ID NO: 6, 29-287 of SEQ ID NO: 7, 34-292 of SEQ ID NO: 8, 34-292 of SEQ ID NO: 9, 56-325 of SEQ ID NO: 10, or 51-308 of SEQ ID NO: 11, or
60% similar to the amino acid sequence 80 to 338 of SEQ ID NO: 1, 69-327 of SEQ ID NO: 2, 150-408 of SEQ ID NO: 3, 105-363 of SEQ ID NO: 4, 66-324 of SEQ ID NO: 5, 32-290 of SEQ ID NO: 6, 29-287 of SEQ ID NO: 7, 34-292 of SEQ ID NO: 8, 34-292 of SEQ ID NO: 9, 56-325 of SEQ ID NO: 10, or 51-308 of SEQ ID NO: 11,

(2) the amino acid sequence comprising the PS is at least

23% identical to the amino acid sequence 343 to 373 of SEQ ID NO: 1, 332-362 of SEQ ID NO: 2, 414-444 of SEQ ID NO: 3, 368-398 of SEQ ID NO: 4, 330-360 of SEQ ID NO: 56, 296-326 of SEQ ID NO: 6, 293-323 of SEQ ID NO: 7, 298-328 of SEQ ID NO: 8, 298-328 of SEQ ID NO: 9, 334-364 of SEQ ID NO: 10, or 317-347 of SEQ ID NO: 11, or
42% similar to the amino acid sequence 343 to 373 of SEQ ID NO: 1, 332-362 of SEQ ID NO: 2, 414-444 of SEQ ID NO: 3, 368-398 of SEQ ID NO: 4, 330-360 of SEQ ID NO: 56, 296-326 of SEQ ID NO: 6, 293-323 of SEQ ID NO: 7, 298-328 of SEQ ID NO: 8, 298-328 of SEQ ID NO: 9, 334-364 of SEQ ID NO: 10, or 317-347 of SEQ ID NO: 11,
and/or

(3) the amino acid sequence comprising the CLD is at least

27% identical to the amino acid sequence 374 to 522 of SEQ ID NO: 1, 363-511 of SEQ ID NO: 2, 445-592 of SEQ ID NO: 3, 399-547 of SEQ ID NO: 4, 361-509 of SEQ ID NO: 5, 327-474 of SEQ ID NO: 6, 324-471 of SEQ ID NO: 7, 329-475 of SEQ ID NO: 8, 329-475 of SEQ ID NO: 9, 365-521 of SEQ ID NO: 10, or 348-507 of SEQ ID NO: 11, or
50% similar to the amino acid sequence 374 to 522 of SEQ ID NO: 1363-511 of SEQ ID NO: 2, 445-592 of SEQ ID NO: 3, 399-547 of SEQ ID NO: 4, 361-509 of SEQ ID NO: 5, 327-474 of SEQ ID NO: 6, 324-471 of SEQ ID NO: 7, 329-475 of SEQ ID NO: 8, 329-475 of SEQ ID NO: 9, 365-521 of SEQ ID NO: 10, or 348-507 of SEQ ID NO: 11.

11. Polypeptide of any one of claims 1 to 12, wherein said polypeptide comprises an amino acid sequence which is at least

70% identical to any one of SEQ ID Nos. 1 to 11, or
85% similar to any one of SEQ ID Nos. 1 to 11.

12. Polynucleotide encoding the polypeptide of any one of claims 2 to 11.

13. Vector comprising the polynucleotide of claim 12.

14. Host cell comprising the polypeptide of any one of claims 1 to 11, the polynucleotide of claim 12, and/or the vector of claim 13.

15. Method for measuring conformational change or activation status of a CDPK polypeptide, comprising the following steps:

(1) cultivating the host cell of claim 14 comprising the polypeptide of any one of claims 1 to 11 under suitable conditions,
(2) optionally adding calcium and/ or applying stress treatment to the host cell, and
(3) measuring FRET occurrence.

**Patentansprüche**

1. Calciumabhängige-Proteinkinase(CDPK)-Polypeptid, umfassend eine variable Domäne (VD), eine Proteinkinasedomäne (PKD), ein Pseudosubstratsegment (PS) und eine calmodulinähnliche Domäne (CLD), wobei die CLD 4 EF-Hand-Motive EF1, EF2, EF3 und EF4 umfasst, wobei

(A) ein Donor-Chromophor zwischen PKD und PS insertiert wird und ein Akzeptor-Chromophor C-terminal von EF4 insertiert wird, oder
(B) ein Akzeptor-Chromophor zwischen PKD und PS insertiert wird und ein Donor-Chromophor C-terminal von EF4 insertiert wird,

wobei das Donor-Chromophor und das Akzeptor-Chromophor ein Förster-Resonanzenergietransfer(FRET)-Paar darstellen.

2. Polypeptid nach Anspruch 1, wobei das Donor-Chromophor und/oder das Akzeptor-Chromophor ein fluoreszierendes Protein (FP) ist.

3. Polypeptid nach Anspruch 1 oder 2, wobei

(a) das Donor-Chromophor ein cyan fluoreszierendes Protein (CFP) ist und das Akzeptor-Chromophor ein gelb fluoreszierendes Protein (YFP) ist,
(b) das Donor-Chromophor ein grün fluoreszierendes Protein (GFP) oder gelb fluoreszierendes Protein (YFP) ist und das Akzeptor-Chromophor ein rot fluoreszierendes Protein (RFP) ist,
(c) das Donor-Chromophor ein cyan fluoreszierendes Protein (CFP) ist und das Akzeptor-Chromophor ein grün fluoreszierendes Protein (GFP) ist,
(d) das Donor-Chromophor ein rot fluoreszierendes Protein (RFP) ist und das Akzeptor-Chromophor ein Nahinfrarot-fluoreszierendes Protein ist, oder
(e) das Donor-Chromophor eine nicht natürlich vorkommende Aminosäure ist und das Akzeptor-Chromophor ein fluoreszierendes Protein ist.

4. Polypeptid nach einem der Ansprüche 1 bis 3, wobei

(i) das Donor-Chromophor ausgewählt ist aus der Gruppe bestehend aus mTurquoise (mT), eCFP, Aquamarine, mTurquoise2 (mT2), mCerulean3, mTFP1 und LUMP, und
das Akzeptor-Chromophor ausgewählt ist aus der Gruppe bestehend aus cpVenus173, eYFP, mVenus, Venus, mCitrine, sEYFP und YPet, oder
(ii) das Donor-Chromophor ausgewählt ist aus der Gruppe bestehend aus cpVenus173, eGFP, NowGFP, Clover, mClover3 und mNeonGreen, und das Akzeptor-Chromophor ausgewählt ist aus der Gruppe bestehend aus mRuby2, mRuby3, mRFP, nKOk und mCherry.

5. Polypeptid nach einem der Ansprüche 1 bis 4, wobei das Donor-Chromophor mT oder eCFP ist und das Akzeptor-Chromophor cpVenus173 ist.

6. Polypeptid nach einem der Ansprüche 1 bis 5, wobei das Donor-Chromophor direkt am C-Terminus der PKD, direkt am N-Terminus des PS oder zwischen dem C-Terminus der PKD und dem N-Terminus des PS insertiert ist.

7. Polypeptid nach einem der Ansprüche 1 bis 6, wobei das Akzeptor-Chromophor direkt am C-Terminus von EF4, am C-Terminus des CDPK-Polypeptids oder zwischen dem C-Terminus von EF4 und dem C-Terminus des CDPK-Polypeptids insertiert ist.

8. Polypeptid nach einem der Ansprüche 1 bis 7, wobei die Aminosäuresequenz, die die PKD, das PS und die CLD der CDPK umfasst, mindestens zu 37 % identisch ist mit der Aminosäuresequenz 80 bis 522 von SEQ ID NO: 1, 69 bis 511 von SEQ ID NO: 2, 150 bis 592 von SEQ ID NO: 3, 105 bis 547 von SEQ ID NO: 4, 66 bis 509 von SEQ ID NO: 5, 32 bis 474 von SEQ ID NO: 6, 29 bis 471 von SEQ ID NO: 7, 34 bis 475 von SEQ ID NO: 8, 34 bis 475 von SEQ ID NO: 9, 56 bis 521 von SEQ ID NO: 10 oder 51 bis 507 von SEQ ID NO: 11.

9. Polypeptid nach einem der Ansprüche 1 bis 8, wobei die Aminosäuresequenz, die die PKD, das PS und die CLD der CDPK umfasst, mindestens zu 54 % ähnlich ist der Aminosäuresequenz 80 bis 522 von SEQ ID NO: 1, 69 bis 511 von SEQ ID NO: 2, 150 bis 592 von SEQ ID NO: 3, 105 bis 547 von SEQ ID NO: 4, 66 bis 509 von SEQ ID NO: 5, 32 bis 474 von SEQ ID NO: 6, 29 bis 471 von SEQ ID NO: 7, 34 bis 475 von SEQ ID NO: 8, 34 bis 475 von SEQ ID NO: 9, 56 bis 521 von SEQ ID NO: 10 oder 51 bis 507 von SEQ ID NO: 11.

10. Polypeptid nach einem der Ansprüche 1 bis 9, wobei

(1) die Aminosäuresequenz, die die PKD umfasst, mindestens

zu 44 % identisch ist mit der Aminosäuresequenz 80 bis 338 von SEQ ID NO: 1, 69 bis 327 von SEQ ID NO: 2, 150 bis 408 von SEQ ID NO: 3, 105 bis 363 von SEQ ID NO: 4, 66 bis 324 von SEQ ID NO: 5, 32 bis 290 von SEQ ID NO: 6, 29 bis 287 von SEQ ID NO: 7, 34 bis 292 von SEQ ID NO: 8, 34 bis 292 von SEQ ID NO: 9, 56 bis 325 von SEQ ID NO: 10 oder 51 bis 308 von SEQ ID NO: 11, oder
zu 60 % ähnlich ist der Aminosäuresequenz 80 bis 338 von SEQ ID NO: 1, 69 bis 327 von SEQ ID NO: 2, 150 bis 408 von SEQ ID NO: 3, 105 bis 363 von SEQ ID NO: 4, 66 bis 324 von SEQ ID NO: 5, 32 bis 290 von SEQ ID NO: 6, 29 bis 287 von SEQ ID NO: 7, 34 bis 292 von SEQ ID NO: 8, 34 bis 292 von SEQ ID NO: 9, 56 bis 325 von SEQ ID NO: 10 oder 51 bis 308 von SEQ ID NO: 11,

(2) die Aminosäuresequenz, die das PS umfasst, mindestens

zu 23 % identisch ist mit der Aminosäuresequenz 343 bis 373 von SEQ ID NO: 1, 332 bis 362 von SEQ ID NO: 2, 414 bis 444 von SEQ ID NO: 3, 368 bis 398 von SEQ ID NO: 4, 330 bis 360 von SEQ ID NO: 56, 296 bis 326 von SEQ ID NO: 6, 293 bis 323 von SEQ ID NO: 7, 298 bis 328 von SEQ ID NO: 8, 298 bis 328 von SEQ ID NO: 9, 334 bis 364 von SEQ ID NO: 10 oder 317 bis 347 von SEQ ID NO: 11, oder
zu 42 % ähnlich ist der Aminosäuresequenz 343 bis 373 von SEQ ID NO: 1, 332 bis 362 von SEQ ID NO: 2, 414 bis 444 von SEQ ID NO: 3, 368 bis 398 von SEQ ID NO: 4, 330 bis 360 von SEQ ID NO: 56, 296 bis 326 von SEQ ID NO: 6, 293 bis 323 von SEQ ID NO: 7, 298 bis 328 von SEQ ID NO: 8, 298 bis 328 von SEQ ID NO: 9, 334 bis 364 von SEQ ID NO: 10 oder 317 bis 347 von SEQ ID NO: 11,
und/oder

(3) die Aminosäuresequenz, die die CLD umfasst, mindestens

zu 27 % identisch ist mit der Aminosäuresequenz 374 bis 522 von SEQ ID NO: 1, 363 bis 511 von SEQ ID NO: 2, 445 bis 592 von SEQ ID NO: 3, 399 bis 547 von SEQ ID NO: 4, 361 bis 509 von SEQ ID NO: 5, 327 bis 474 von SEQ ID NO: 6, 324 bis 471 von SEQ ID NO: 7, 329 bis 475 von SEQ ID NO: 8, 329 bis 475 von SEQ ID NO: 9, 365 bis 521 von SEQ ID NO: 10 oder 348 bis 507 von SEQ ID NO: 11, oder
zu 50 % ähnlich ist der Aminosäuresequenz 374 bis 522 von SEQ ID NO: 1, 363 bis 511 von SEQ ID NO: 2, 445 bis 592 von SEQ ID NO: 3, 399 bis 547 von SEQ ID NO: 4, 361 bis 509 von SEQ ID NO: 5, 327 bis 474 von SEQ ID NO: 6, 324 bis 471 von SEQ ID NO: 7, 329 bis 475 von SEQ ID NO: 8, 329 bis 475 von SEQ ID NO: 9, 365 bis 521 von SEQ ID NO: 10 oder 348 bis 507 von SEQ ID NO: 11.

11. Polypeptid nach einem der Ansprüche 1 bis 12, wobei das Polypeptid eine Aminosäuresequenz umfasst, die mindestens

zu 70 % identisch mit einer der SEQ ID NO: 1 bis 11 ist, oder
zu 85 % ähnlich einer der SEQ ID NO: 1 bis 11 ist.

12. Polynukleotid, kodierend für das Polypeptid nach einem der Ansprüche 2 bis 11.

**13.** Vektor, umfassend das Polynukleotid nach Anspruch 12.

**14.** Wirtszelle, umfassend das Polypeptid nach einem der Ansprüche 1 bis 11, das Polynukleotid nach Anspruch 12, und/oder den Vektor nach Anspruch 13.

**15.** Verfahren zum Messen der Konformationsänderung oder des Aktivierungsstatus eines CDPK-Polypeptids, umfassend die folgenden Schritte:

(1) Kultivieren der Wirtszelle nach Anspruch 14, die das Polypeptid nach einem der Ansprüche 1 bis 11 umfasst, unter geeigneten Bedingungen,
(2) gegebenenfalls Zugeben von Calcium und/oder Anwenden einer Stressbehandlung auf die Wirtszelle, und
(3) Messen des Auftretens von FRET.

**Revendications**

**1.** Polypeptide de protéine kinase dépendant du calcium (CDPK) comprenant un domaine variable (VD), un domaine de protéine kinase (PKD), un segment de pseudo-substrat (PS) et un domaine de type calmoduline (CLD), ledit CLD comprenant 4 motifs de mains EF EF1, EF2, EF3 et EF4,
dans lequel

(A) un chromophore donneur est inséré entre PKD et PS et un chromophore accepteur est inséré à l'extrémité C-terminale de EF4, ou
(B) un chromophore accepteur est inséré entre PKD et PS et un chromophore donneur est inséré à l'extrémité C-terminale de EF4,

dans lequel ledit chromophore donneur et ledit chromophore accepteur représentent une paire de transfert d'énergie par résonance de Forster (FRET).

**2.** Polypeptide selon la revendication 1, dans lequel ledit chromophore donneur et/ou ledit chromophore accepteur est/sont une protéine fluorescente (FP).

**3.** Polypeptide selon la revendication 1 ou 2, dans lequel

(a) ledit chromophore donneur est un FP cyan (CFP) et ledit chromophore accepteur est un FP jaune (YFP),
(b) ledit chromophore donneur est un FP vert (GFP) ou un FP jaune (YFP) et ledit chromophore accepteur est un FP rouge (RFP),
(c) ledit chromophore donneur est un FP cyan (CFP) et ledit chromophore accepteur est un FP vert (GFP),
(d) ledit chromophore donneur est un FP rouge (RFP) et ledit chromophore accepteur est un FP proche-infrarouge, ou
(e) ledit chromophore donneur est un acide aminé d'origine non naturelle et ledit chromophore accepteur est un FP.

**4.** Polypeptide selon l'une quelconque des revendications 1 à 3, dans lequel

(i) ledit chromophore donneur est choisi dans le groupe constitué par mTurquoise (mT), eCFP, Aquamarine, mTurquoise2 (mT2), mCerulean3, mTFP1 et LUMP, et
ledit chromophore accepteur est choisi dans le groupe constitué par cpVenus173, eYFP, mVenus, Venus, mCitrine, sEYFP et YPet, ou
(ii) ledit chromophore donneur est choisi dans le groupe constitué par cpVenus173, eGFP, NowGFP, Clover, mClover3 et mNeonGreen, et ledit chromophore accepteur est choisi dans le groupe constitué par mRuby2, mRuby3, mRFP, nKOk et mCherry.

**5.** Polypeptide selon l'une quelconque des revendications 1 à 4, dans lequel ledit chromophore donneur est mT ou eCFP et ledit chromophore accepteur est cpVenus173.

**6.** Polypeptide selon l'une quelconque des revendications 1 à 5, dans lequel ledit chromophore donneur est inséré directement à l'extrémité C-terminale du PKD, directement à l'extrémité N-terminale du PS ou entre l'extrémité C-terminale du PKD et l'extrémité N-terminale du PS.

7. Polypeptide selon l'une quelconque des revendications 1 à 6, dans lequel ledit chromophore accepteur est inséré directement à l'extrémité C-terminale de EF4, à l'extrémité C-terminale du polypeptide CDPK ou entre l'extrémité C-terminale de EF4 et l'extrémité C-terminale du polypeptide CDPK.

8. Polypeptide selon l'une quelconque des revendications 1 à 7, dans lequel la séquence d'acides aminés comprenant le PKD, le PS et le CLD du CDPK est d'au moins 37 % identique à la séquence d'acides aminés 80 à 522 de la SEQ ID NO : 1, 69-511 de SEQ ID NO : 2, 150-592 de SEQ ID NO : 3, 105-547 de SEQ ID NO : 4, 66-509 de SEQ ID NO : 5, 32-474 de SEQ ID NO : 6, 29-471 de SEQ ID NO : 7, 34-475 de SEQ ID NO : 8, 34-475 de SEQ ID NO : 9, 56-521 de SEQ ID NO : 10, ou 51-507 de SEQ ID NO : 11.

9. Polypeptide selon l'une quelconque des revendications 1 à 8, dans lequel la séquence d'acides aminés comprenant le PKD, le PS et le CLD du CDPK est d'au moins 54 % similaire à la séquence d'acides aminés 80 à 522 de la SEQ ID NO : 1, 69-511 de SEQ ID NO : 2, 150-592 de SEQ ID NO : 3, 105-547 de SEQ ID NO : 4, 66-509 de SEQ ID NO : 5, 32-474 de SEQ ID NO : 6, 29-471 de SEQ ID NO : 7, 34-475 de SEQ ID NO : 8, 34-475 de SEQ ID NO : 9, 56-521 de SEQ ID NO : 10, ou 51-507 de SEQ ID NO : 11.

10. Polypeptide selon l'une quelconque des revendications 1 à 9, dans lequel

(1) la séquence d'acides aminés comprenant le PKD est d'au moins

44 % identique à la séquence d'acides aminés 80 à 338 de la SEQ ID NO : 1, 69-327 de SEQ ID NO : 2, 150-408 de SEQ ID NO : 3, 105-363 de SEQ ID NO : 4, 66-324 de SEQ ID NO : 5, 32-290 de SEQ ID NO : 6, 29-287 de SEQ ID NO : 7, 34-292 de SEQ ID NO : 8, 34-292 de SEQ ID NO : 9, 56-325 de SEQ ID NO : 10, ou 51-308 de SEQ ID NO : 11 ou
60 % similaire à la séquence d'acides aminés 80 à 338 de la SEQ ID NO : 1, 69-327 de SEQ ID NO : 2, 150-408 de SEQ ID NO : 3, 105-363 de SEQ ID NO : 4, 66-324 de SEQ ID NO : 5, 32-290 de SEQ ID NO : 6, 29-287 de SEQ ID NO : 7, 34-292 de SEQ ID NO : 8, 34-292 de SEQ ID NO : 9, 56-325 de SEQ ID NO : 10, ou 51-308 de SEQ ID NO : 11,

(2) la séquence d'acides aminés comprenant le PS est d'au moins

23 % identique à la séquence d'acides aminés 343 à 373 de la SEQ ID NO : 1, 332-362 de SEQ ID NO : 2, 414-444 de SEQ ID NO : 3, 368-398 de SEQ ID NO : 4, 330-360 de SEQ ID NO : 56, 296-326 de SEQ ID NO : 6, 293-323 de SEQ ID NO : 7, 298-328 de SEQ ID NO : 8, 298-328 de SEQ ID NO : 9, 334-364 de SEQ ID NO : 10, ou 317-347 de SEQ ID NO : 11 ou
42 % similaire à la séquence d'acides aminés 343 à 373 de la SEQ ID NO : 1, 332-362 de SEQ ID NO : 2, 414-444 de SEQ ID NO : 3, 368-398 de SEQ ID NO : 4, 330-360 de SEQ ID NO : 56, 296-326 de SEQ ID NO : 6, 293-323 de SEQ ID NO : 7, 298-328 de SEQ ID NO : 8, 298-328 de SEQ ID NO : 9, 334-364 de SEQ ID NO : 10, ou 317-347 de SEQ ID NO : 11,
et/ou

(3) la séquence d'acides aminés comprenant le CLD est d'au moins

27 % identique à la séquence d'acides aminés 374 à 522 de la SEQ ID NO : 1, 363-511 de SEQ ID NO : 2, 445-592 de SEQ ID NO : 3, 399-547 de SEQ ID NO : 4, 361-509 de SEQ ID NO : 5, 327-474 de SEQ ID NO : 6, 324-471 de SEQ ID NO : 7, 329-475 de SEQ ID NO : 8, 329-475 de SEQ ID NO : 9, 365-521 de SEQ ID NO : 10, ou 348-507 de SEQ ID NO : 11 ou
50 % similaire à la séquence d'acides aminés 374 à 522 de la SEQ ID NO : 1, 363-511 de SEQ ID NO : 2, 445-592 de SEQ ID NO : 3, 399-547 de SEQ ID NO : 4, 361-509 de SEQ ID NO : 5, 327-474 de SEQ ID NO : 6, 324-471 de SEQ ID NO : 7, 329-475 de SEQ ID NO : 8, 329-475 de SEQ ID NO : 9, 365-521 de SEQ ID NO : 10, ou 348-507 de SEQ ID NO : 11.

11. Polypeptide selon l'une quelconque des revendications 1 à 12, dans lequel ledit polypeptide comprend une séquence d'acides aminés qui est d'au moins

70 % identique à l'une quelconque des SEQ ID n° 1 à 11, ou

85 % similaire à l'une quelconque des SEQ ID n° 1 à 11.

12. Polynucléotide codant pour le polypeptide selon l'une quelconque des revendications 2 à 11.

13. Vecteur comprenant le polynucléotide selon la revendication 12.

14. Cellule hôte comprenant le polypeptide selon l'une quelconque des revendications 1 à 11, le polynucléotide selon la revendication 12 et/ou le vecteur selon la revendication 13.

15. Méthode pour mesurer le changement conformationnel ou l'état d'activation d'un polypeptide CDPK, comprenant les étapes suivantes :

(1) la culture de la cellule hôte selon la revendication 14 comprenant le polypeptide selon l'une quelconque des revendications 1 à 11 dans des conditions appropriées,
(2) l'ajout facultatif de calcium et/ou l'application d'un traitement de stress à la cellule hôte, et
(3) la mesure de l'occurrence de FRET.

**Figure 1**

**Figure 2**

**Figure 3**

| Kinase activity | | | | |
|---|---|---|---|---|
| CPK | K50 $Ca^{2+}$ (nM) | core activity (%) | Hillslope | n |
| CPK21 | 449 +/- 90 | 11 +/- 6 | 3.1 | 4 |
| CPK23 | 1864 +/- 156 | 39 +/- 3 | 3.2 | 4 |
| Conformational change | | | | |
| CPK-FRET | EC50 $Ca^{2+}$ (nM) | $\Delta$FRETmax (%) | Hillslope | n |
| CPK21D204A | 832 +/- 53 | 27 +/- 1 | 2.1 | 16 |
| CPK23D193A | 1391 +/- 188 | 10 +/- 1 | 3.5 | 7 |

**Figure 4**

**Figure 5**

**Figure 6**

| CPK | Kinase activity | | | |
|---|---|---|---|---|
| | K50 Ca$^{2+}$ (nM) | core activity (%) | Hillslope | n |
| CPK21I373S | 1111 +/- 47 | 28 +/- 7 | 12.2 | 3 |
| CPK21I373D | 1622 +/- 478 | 9 +/- 13 | 4.9 | 2 |
| CPK23S362I | 1229 +/- 39 | 37 +/- 6 | 5.0 | 3 |
| CPK23S362D | not applicable | | | 2 |
| | Conformational change | | | |
| CPK-FRET | EC50 Ca$^{2+}$ (nM) | Δ FRETmax (%) | Hillslope | |
| CPK21D204A-I373S | 925 +/- 153 | 31 +/- 3 | 7.0 | 4 |
| CPK21D204A-I373D | 1701 +/- 294 | 16 +/- 1 | 2.8 | 5 |

**Figure 7**

EP 3 844 270 B1

38

**Figure 8**

**Figure 9**

Figure 10

| CPK | Kinase activity | | | |
|---|---|---|---|---|
| | K50 Ca$^{2+}$ (nM) | core activity (%) | Hillslope | n |
| CPK23CLD21 | 1249 +/- 35 | 48 +/- 2 | 19.8 | 3 |
| CPK23CLD21-S362I | 951 +/- 32 | 28 +/- 5 | 3.6 | 3 |
| CPK-FRET | Conformational change | | | |
| | EC50 Ca$^{2+}$ (nM) | ΔFRETmax (%) | Hillslope | n |
| CPK23D193A-CLD21 | 1458 +/- 163 | 16 +/- 2 | 2.6 | 3 |
| CPK23D193A-S362I-CLD21 | 879 +/- 105 | 30 +/- 1 | 1.9 | 4 |

**Figure 11**

**Figure 12**

**Figure 13**

**Figure 14**

**Figure 15**

**Figure 16**

**Figure 17**

**Figure 18**

Figure 19

| CPK-FRET | Kinase activity | | | n |
|---|---|---|---|---|
| | K50 Ca$^{2+}$ (nM) | core activity (%) | Hillslope | |
| CPK21 | not applicable | | | 2 |
| | Conformational change | | | |
| CPK-FRET | EC50 Ca$^{2+}$ (nM) | $\Delta$ FRETmax (%) | Hillslope | |
| CPK21 | 1517 +/- 261 | 27 +/- 3 | 1.7 | 4 |
| CPK23 | 1533 +/- 67 | 9 +/- 1 | 3.1 | 5 |

**Figure 20**

**Figure 21**

| CPK-FRET | EC50 Ca$^{2+}$ (nM) | Conformational change | | |
|---|---|---|---|---|
| | | ΔFRETmax (%) | Hillslope | n |
| CPK23S362I | 1389 +/- 114 | 9 +/- 1 | 2.6 | 2 |
| CPK23S362D | 1271 +/- 128 | 11 | 4.7 | 4 |

**Figure 22**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5525711 A
- US 4711955 A
- US 5792608 A
- EP 302175 A

### Non-patent literature cited in the description

- **KUDLA et al.** *New Phytol,* 2018
- **SCHULZ et al.** *Plant Physiol,* 2013, vol. 163, 523-530
- **GEIGER et al.** *Sci Signal,* 2011, vol. 4, ra32
- **GEIGER et al.** *Proc Natl Acad Sci U S A,* 2010, vol. 107, 8023-8028
- **DUBIELLA et al.** *Proc Natl Acad Sci USA,* 2013, vol. 110, 8744-8749
- **BOUDSOCQ et al.** *Nature,* 2010, vol. 464, 418-422
- **LIU et al.** *Nature,* 2017, vol. 545, 311-316
- **GUTERMUTH et al.** *New Phytologist,* 2018, vol. 218, 1089-1105
- **GUTERMUTH et al.** *Plant Cell,* 2013, vol. 25, 4525-4543
- **FOROUTAN et al.** *Clin Exp Vaccine Res,* 2018, vol. 7, 24-36
- **OJO et al.** *Nat Struct Mol Biol,* 2010, vol. 17, 602-607
- **BILLKER et al.** *Cell Host Microbe,* 2009, vol. 5, 612-622
- **KATO et al.** *Nat Chem Biol,* 2008, vol. 4, 347-356
- **SIMEUNOVIC et al.** *J Exp Bot,* 2016, vol. 67, 3855-3872
- **LIESE et al.** *Biochim Biophys Acta,* 2013, vol. 1833, 1582-1589
- **BENDER et al.** *Biochem J,* 2018, vol. 475, 207
- **WERNIMONT et al.** *Nat Struct Mol Biol,* 2010, vol. 17, 596-601
- **WERNIMONT et al.** *Proteins,* 2011, vol. 79, 803-820
- **HAMEL et al.** *Trends Plant Sci,* 2014, vol. 19 (2), 79-89
- **DEPRY et al.** *Methods Mol Biol,* 2011, vol. 756, 285-294
- **BAJAR et al.** *Sensors,* 2016, vol. 16, 1488
- **KO et al.** *RSC Advances,* 2016, vol. 6 (82), 78661-78668
- **GAMPER.** *Nucleic Acids Research,* 2000, vol. 28, 4332-4339
- **FRANZ et al.** *Mol Plant,* 2011, vol. 4 (1), 83-96
- **ZHAO et al.** *Science,* 2011, vol. 333, 1888-1891
- **INGRAM et al.** *PNAS,* 2015, E4675-E4984
- **WAADT et al.** *eLife,* 2014, vol. 3, e01739
- **GOEDHARDT et al.** *Nat Methods,* 2010, vol. 7, 137-139
- **NAGAI et al.** *PNAS,* 2004, vol. 101, 10554-10559
- **WITTE et al.** *Plant Mol Biol,* 2004, vol. 55, 135-147
- **KIRCHNER et al.** *PLoS ONE,* 2017, vol. 12, e0185429
- **WENER et al.** *Gene,* 1994, vol. 151, 119-123
- **HEIM et al.** *Curr Biol,* 1996, vol. 6, 178-182
- **SCHMIDT et al.** *Nat Protoc,* 2007, vol. 2, 1528-1535
- The UniProt Consortium. *Nucleic Acid Res,* 2017, vol. 45, D158-D169
- **CROOKS et al.** *Genome Res,* 2004, vol. 14, 1188-1190
- **SCHNEIDER et al.** *Nucleic Acid Res,* 1990, vol. 18, 6097-6100
- **PATTON et al.** *Cell Calcium,* 2004, vol. 35, 427-431
- **FRANZ et al.** *Mol Plant,* 2011, vol. 79, 803-820
- **WU et al.** *Plant Methods,* 2009, vol. 5, 16
- **MA et al.** *Plant Mol Biol,* 2007, vol. 65, 511-518
- **DUBIELLA et al.** *PNAS,* 2013, vol. 110, 8744-8749
- **SCHMIDT et al.** *Mol Syst Biol,* 2011, vol. 7, 510
- **SCHROEDER et al.** *Anall Chem,* 2004, vol. 76, 3590-3598
- **MENZ et al.** *Plant J,* 2016, vol. 88, 717-734